# EUROPEAN PATENT SPECIFICATION

(11) **EP 1 467 720 B1**
(45) Date of publication and mention of the grant of the patent: **01.04.2009**
(21) Application number: 03701595.5
(22) Date of filing: 23.01.2003
(51) Int. Cl.: A61K 9/70, C12N 15/89

(54) **DNA DOSAGE FORMS**
DARREICHUNGSFORMEN FÜR DNA
FORMES POSOLOGIQUES A BASE D'ADN

(30) Priority: 25.01.2002 GB 0201736
(43) Date of publication of application: 20.10.2004
(73) Proprietor: GLAXO GROUP LIMITED, Greenford, Middlesex UB6 0NN (GB)
(72) Inventor: CATCHPOLE, Ian Richard, c/o GlaxoSmithKline, Stevenage, Hertfordshire SG1 2NY (GB)
(74) Representative: Easeman, Richard Lewis
(86) International application number: PCT/GB2003/000282
(87) International publication number: WO 2003/061636

(56) References cited:
- EP-A- 1 138 337
- WO-A-96/13277
- WO-A-97/40839

## Description

The present invention relates to efficient devices for administration of DNA based pharmaceutical agents into mammalian skin. In a particularly preferred aspect of the present invention there is provided devices for administration of DNA vaccines into the skin of the mammal, and preferably into human skin. The present invention provides a microneedle DNA pharmaceutical agent delivery device having at least one skin-piercing element which comprises a support member coated with a solid reservoir medium containing, in solid solution or suspension within it, the DNA pharmaceutical agent, and a stabilising agent that inhibits the degradative effects of free radicals. Preferably the solid pharmaceutical reservoir medium coated onto such devices is a polyol, preferably being a polyol in an amorphous state (such as a glass). In the context of the present invention the free radical stabilising agent is preferably a free radical scavenger and/or a metal ion chelator. Most preferably the DNA vaccine devices of the present invention comprise a DNA plasmid, a free radical scavenger, and a metal ion chelator, in solid solution within a glassy sugar reservoir medium, coated onto a support member. The DNA devices of the present invention are storage stable, and only substantially release the DNA pharmaceutical after penetration of the skin piercing portion into the skin. The DNA pharmaceutical delivery devices are proportioned such that agent is delivered into defined layers of the skin, and preferred delivery devices comprise skin-piercing portions that deliver the pharmaceutical agent into the epithelium or the dermis. Preferred reservoir media comprise sugars, and in particular stabilising sugars that form a glass such as lactose, raffinose, trehalose, glucose or sucrose. Furthermore, vaccine delivery devices for administration of vaccines into the skin are provided, methods of their manufacture, and their use in medicine.

The skin represents a significant barrier to external agents. A summary of human skin is provided in Dorland's Illustrated Medical Dictionary, 28^{th} Edition. Starting from the external layers, working inwards, the skin comprises the epithelium comprising the stratum corneum, the viable epithelium, and underlying the epithelium is the dermis. The epithelium consists of five layers: Stratum corneum, Stratum lucidium, Stratum granulosum, Stratum spinosum, and Stratum basale. The *epithelium* (including all five layers) is the outermost non-vascular layer of the skin, and varies between 0.07 and 0.12 mm thick (70-120 µm). The epithelium is populated with keratinocytes, a cell that produces keratin and constitutes 95% of the dedicated epidermal cells. The other 5% of cells are melanocytes. The underlying dermis is normally found within a range of 0.3 to about 3 mm beneath the surface of the stratum corneum, and contains sweat glands, hair follicles, nerve endings and blood vessels.

The stratum corneum dominates the skin permeability barrier and consists of a few dozen horny, keratinised epithelium layers. The narrow interstices between the dead or dying keratinocytes in this region are filled with crystalline lipid multilamellae. These efficiently seal the interstices between the skin or body interior and the surroundings by providing a hydrophobic barrier to entry by hydrophilic molecules. The stratum corneum being in the range of 30-70 µm thick.

Langerhans cells are found throughout the basal granular layer of the epithelium (stratum spinosum and stratum granulosum, (Small Animal Dermatology - Third Edition, Muller - Kirk - Scott, Ed: Saunders (1983)) and are considered to play an important role in the immune system's initial defence against invading organisms. This layer of the skin therefore represents a suitable target zone for certain types of vaccine.

Conventional modes for administration of pharmaceutical agents into or across the skin, most commonly by hypodermic needle and syringe, are associated with numerous disadvantages. Such disadvantages include pain, the requirement for trained professionals to administer the agent, and also the risk of needle-stick injuries to the administrator with the accompanying risk of infection with a blood born disease. As such, there is a need to improve the method of administration of all types of pharmaceutical into or through the skin.

A number of alternative approaches have been described in order to overcome the problems of administering agent across the stratum corneum, including various designs of skin patches. Examples of skin patches which deliver agent through the skin without physically penetrating the stratum corneum layer include that described in WO 98/20734 and WO 99/43350. Other approaches where the skin is not physically punctured include electrotransport, or iontophoretic devices where the passage of agent is enhanced by the application of an electrical current into the skin. Many such devices are described in the literature (examples of which include US 6,083,190; US 6,057,374; US 5,995,869; US 5,622,530, WO 00/44438). Potential disadvantages of these types of non-penetration patches include the induction of significant sensitisation and discomfort during administration of the agent, and very poor uptake of antigen across the intact stratum corneum.

Other patches involving physical disruption or penetration of the skin have been described. Devices comprising liquid or solid reservoirs containing agent and a metal microblade patch have been described wherein the microblades physically cut through the stratum corneum to create pathways through which the agent can enter the epithelium. Such devices are described in WO 97/48440, WO 97/48442, WO 98/28037, WO 99/29298, WO 99/29364, WO 99/29365, WO 00/05339, WO 00/05166, and WO 00/16833. Other devices involving puncturing of the skin include US 5,279,544, US 5,250,023 and US 3,964,482.

Solid dosage forms comprising a pharmaceutical agents and a stabilising polyol, such as a sugar wherein the dosage forms are in the form of powders and trocars are described in WO 96/03978.

Supercoiled DNA in pharmaceutical preparations are known to degrade over time resulting in the loss of the supercoiled structure and associated formation of open circle or linear DNA structures (Evans et al., 2000, Journal of Pharmaceutical Sciences, 89(1), 76-87; WO 97/40839). One mechanism by which this chain scission reaction may occur is oxidation of the DNA by free hydroxyl radicals produced from dissolved oxygen in the DNA solutions, a process that is catalysed by metal ions. The free radical formation reaction may be catalysed by several transition metal ions, the most common of which, however, are iron and copper ions (Fe⁺³, Fe⁺², Cu⁺² or Cu⁺¹; Evans *et al. supra*).

It has been shown that removal of trace metal ions from supercoiled DNA containing solutions with metal ion chelators, and/or mopping up free radicals in solution by non-reducing free radical scavengers stabilises the DNA in the supercoiled form and protects the DNA from oxidation (WO 97/40839).

Plasmid based delivery of genes, particularly for immunisation or gene therapy purposes is known. For example, administration of naked DNA by injection into mouse muscle is outlined in WO90/11092. Johnston et al WO 91/07487 describe methods of transferring a gene to veterbrate cells, by the use of microprojectiles that have been coated with a polynucleotide encoding a gene of interest, and accelerating the microparticles such that the microparticles can penetrate the target cell.

DNA vaccines usually consist of a bacterial plasmid vector into which is inserted a strong viral promoter, the gene of interest which encodes for an antigenic peptide and a polyadenylation/transcriptional termination sequences. The gene of interest may encode a full protein or simply an antigenic peptide sequence relating to the pathogen, tumour or other agent which is intended to be protected against. The plasmid can be grown in bacteria, such as for example *E.coli* and then isolated and prepared in an appropriate medium, depending upon the intended route of administration, before being administered to the host. Following administration the plasmid is taken up by cells of the host where the encoded protein or peptide is produced. The plasmid vector will preferably be made without an origin of replication which is functional in eukaryotic cells, in order to prevent plasmid replication in the mammalian host and integration within chromosomal DNA of the animal concerned. Information in relation to DNA vaccination is provided in Donnelly et al "DNA vaccines" Ann. Rev Immunol. 1997 15: 617-648. DNA vaccination may also encompass techniques such as administration of viral or bacterial vectors, which encode and express the heterogenous vaccine antigen.

The present invention overcomes the problems of the prior art and provides a device which is capable of administering and releasing the DNA agents efficiently into the skin, and also in which the DNA is stabilised such that it is released in its supercoiled form.

### DESCRIPTION OF THE FIGURES.

**FIG 1****.** shows the plasmids used in this study
   A. pEGFP-C1, B. pGL3CMV, C. pVAC1.ova
**FIG 2**. shows 0.8% agarose gel electrophoresis using an E-gel, for analysis of supercoiled plasmid DNA, pEGFP-C1, after coating onto and immediate elution from sewing needles.
**FIG 3****.** shows 1.2% agarose gel electrophoresis using an E-gel, for analysis of supercoiled plasmid DNA, pEGFP-C1, after coating onto and elution from sewing needles.
**FIG. 4** shows 1.2% agarose gel electrophoresis using an E-gel, for analysis of supercoiled plasmid DNA, pGL3CMV, after coating onto and elution from sewing needles stored for varying time periods at 4°C.
**FIG. 5** shows 1% agarose gel electrophoresis, in the absence of ethidium bromide, (EtBr), for analysis of supercoiled plasmid DNA, pGL3CMV, after coating onto sewing needles.
**FIG. 6** shows 1.2% agarose gel electrophoresis using an E-gel, for analysis of supercoiled plasmid DNA, pGL3CMV, after coating onto and immediate elution from sewing needles or 30G hypodermic needles.
**FIG. 7** shows 1% agarose gel electrophoresis, in the absence of ethidium bromide, (EtBr), for analysis of supercoiled plasmid DNA, pVaclova, (resuspended in a variety of different formulations at 5ug/ul), after coating onto sewing or hypodermic needles of different gauges and 'release' into agarose.
**FIG. 8** shows 1% agarose gel electrophoresis, in the absence of ethidium bromide, (EtBr), for analysis of supercoiled plasmid DNA, pVaclova,
**FIG. 9** shows comparative luciferase activity, (48 hours post transfection), derived from plasmid pGL3CMV, delivered either intradermally, (ID, Fig. 9A), or intramuscularly, (IM, Fig. 9B), to mice, from DNA coated 30G hypodermic needles compared to controls of standard ID and standard IM delivery in saline.
**FIG 10** shows comparative luciferase activity, (48 hours post transfection), derived from plasmid pGL3CMV, delivered intradermally, (ID), to mice, from DNA coated 30G hypodermic needles and DNA coated sowing needles, compared to controls of standard ID delivery in saline.
**FIG 11** shows comparative luciferase activity, (24 hours post transfection), derived from plasmid pGL3CMV, delivered either intradermally, (ID, Fig. 11A), or intramuscularly, (IM, Fig. 11B), to mice, from pairs of DNA coated sowing needles and compared to DNA coated 2 needle array electrodes plus electroporation.
**FIG 12** shows comparative luciferase activity, (24 hours post transfection), derived from plasmid pGL3CMV, delivered intradermally, (ID), to mice, from DNA coated microneedles plus electroporation through calliper electrodes.
**FIG 13** shows a graphical plot of the percentage of supercoiled plasmid, (%ccc), both monomeric, (%cccmon), and dimeric, (%cccdim), plasmid forms; after coating and lyophilization onto sowing needles and storage at 37°C. The plasmid formulations used contain varying amounts of sugars: FIG 13A: 5% Sucrose, FIG 13B: 10% Sucrose, FIG 13C: 17.5% Sucrose, FIG 13D: 40% Sucrose, FIG 13E: 40% Trehalose, FIG 13F: 40% Glucose.
**FIG 14** shows differential scanning calorimetry, (DSC), data, for plasmid DNA, (10mg/ml), formulations in 40% sucrose. Fig 14A & B: formulations also contain: 100mM TrisHCl pH8.0, 1mM EDTA, 10mM methionine and 2.9% ethanol; Fig 14A & C represent a 24 hour lyophilization cycle; Fig 14B & D represent a 1 hour lyophilization cycle.
**FIG 15** shows polarized light microscopy data, for plasmid DNA, (10mg/ml), formulations in 40% sucrose. Fig 15A: formulations also contain: 100mM TrisHCl pH8.0, 1mM EDTA, 10mM methionine and 2.9% ethanol, Fig15C: only contains 40% sucrose and Fig 15D: shows crystals of the excipients described in the formulation shown in Fig 15A. 1AM, 2AM & 3AM represent a 24 hour lyophilization cycle, whereas 1ST, 2ST & 3ST represent a 1 hour lyophilization cycle.
**FIG 16** shows polarized light microscopy data, for lyophilisized plasmid DNA, (10mg/ml), formulations in sugars and polyols, which also contain: 100mM TrisHCl pH8.0, 1mM EDTA, 10mM methionine and 2.9% ethanol. Fig 16A, sample 1: 40% w/v ficoll, sample 2:20% w/v dextran, sample 3:40% w/v sucrose, sample 4: 20% w/v maltotriose. Fig 16B, sample 5:20% w/v lactose, sample 6:30% w/v maltose, sample 7: 40% w/v glucose, sample 8:40% w/v trehalose.

The present invention provides a microneedle DNA-based pharmaceutical agent delivery device having at least one skin-piercing microneedle which comprises a support member coated with a solid reservoir medium containing the DNA pharmaceutical agent, and a stabilising agent that inhibits the degradative effects of free radicals. Alternatively, the skin piercing microneedle may consist of the solid DNA pharmaceutical agent reservoir medium without the support member.

Certain embodiments of the devices described herein also have the significant advantage of being stored at room temperature thus reducing logistic costs and releasing valuable refrigerator space for other products.

The skin piercing microneedles, formed by coating the support members with the solid reservoir medium containing the agent to be delivered, after coating with the reservoir medium onto the support member, are long enough and sharp enough to pierce the stratum corneum of the skin. Once the pharmaceutical agent delivery device has been administered to the surface of the skin, and the coated skin-piercing member or microneedle has pierced through the stratum corneum, the solid reservoir medium biodegrades thereby releasing the agent into the skin underlying the stratum corneum.

DNA vaccine delivery devices form a preferred aspect of the present invention. In such applications the agent to be delivered is a polynucleotide that encodes an antigen or antigens derivable from a pathogen such as micro-organisms or viruses, or may be a self antigen in the case of a cancer vaccine or other self antigen.

The support members, which when coated with reservoir medium to form the skin piercing microneedles of the devices of the present invention, may be made of almost any material which can be used to create a protrusion that is strong enough to withstand piercing the stratum corneum, and which is safe for the purpose. For example the protrusions may be made of a metal, such as pharmaceutical grade stainless steel, gold or titanium or other such metal used in prostheses, alloys of these or other metals; ceramics, semi-conductors, silicon, polymers, plastics, glasses or composites.

The delivery devices may be in the form of a single needle, trocar or cannula, or may comprise multiple skin piercing elements in the form of a patch.

The patches of the present invention generally comprise a backing plate or supportive structure from which depend a plurality of piercing protrusions such as microneedles or microblades. The piercing protrusions themselves may take many forms, and may be solid or hollow, and as such may be in the form of a solid needle or blade (such as the microblade aspects and designs described in McAllister et al., Annu. Rev. Biomed. Eng., 2000, 2, 289-313; Henry et al., Journal of Pharmaceutical Sciences, 1998, 87, 8, 922-925; Kaushik et al., Anesth. Analg., 2001, 92, 502-504; McAllister et al., Proceed. Int'l. Symp. Control. Rel. Bioact. Mater., 26, (1999), Controlled Release Society, Inc., 192-193; WO 99/64580; WO 97/48440; WO 97/48442; WO 98/28037; WO 99/29364; WO 99/29365; US 5,879,326. Alternatively the piercing protrusions may be in the form of a microcannula having a hollow central bore. In this last embodiment, the central bore may extend through the needle to form a channel communicating with both sides of the microneedle device (EP 0 796 128 B1). Solid needles and microblades are preferred.

The length of the skin-piercing microneedle for administration of the DNA into the skin may be varied depending on which anatomical location the patch is to be administered and which layer of skin it is desired to administer the pharmaceutical agent in the vaccinee species. Typically between 1µm to 3mm, preferably between 1 µm and 1mm, preferably between 50µm and 600µm, and more preferably between 100 and 400µm. The length of the skin-piercing microneedle may be selected according to the site chosen for targeting delivery of the agent, namely, preferably, the dermis and most preferably the epidermis. The support members of the devices of the present invention may be take the form of, and be manufactured by the methods described in US 5,879,326, WO 97/48440, WO 97/48442, WO 98/28037, WO 99/29298, WO 99/29364, WO 99/29365, WO 99/64580, WO 00/05339, WO 00/05166, or WO 00/16833; or McAllister et al., Annu. Rev. Biomed. Eng., 2000, 2, 289-313; Henry et al., Journal of Pharmaceutical Sciences, 1998, 87, 8, 922-925; Kaushik et al., Anesth. Analg., 2001, 92, 502-504; McAllister et al., Proceed. Int'l. Symp. Control. Rel. Bioact. Mater., 26, (1999); Controlled Release Society, Inc., 192-193.

The most preferred microblade devices to be coated with the pharmaceutical agent reservoir medium to form devices of the present invention are described in WO 99 48440 and Henry et al., Journal of Pharmaceutical Sciences, 1998, 87, 8, 922-925.

The devices of the present invention preferably comprise a plurality of skin-piercing microneedles, preferably up to 1000 microneedles per device, more preferably up to 500 skin-piercing microneedles per device.

Where the piercing protrusion is solid, it may flat or may have a circular or polygonal cross section. The protrusions can have straight or tapered shafts and may be flat or circular, or other polygonal shape, in cross section. For example, the microblades may have a curved blade or be formed into a V-section groove. Alternatively the protrusions may have more complex shapes to enhance adherence and fluid dynamics such as a five pointed star.

The skin-piercing microneedles may be integral with the backing plate or may be attached thereto. In the case where the protrusions may be attached to the plate, the piercing protrusion may be formed of the reservoir medium. Such devices may be made by formed by drawing or extruding a molten reservoir medium containing the agent into fine points. For instance, molten reservoir medium could be cast directly onto a backing plate through a multipore head, where the hot extrudate cools and sticks to the plate. When you draw back the extrudate a series of pointed ends is formed.

As a general feature of any piercing protrusion shape, in order to improve reservoir adherence after coating, the surface of the protrusion may be textured. For example, the surface may be coarse grained, rippled or ribbed. In addition, solid microblades may further comprise through holes, such that the reservoir may dry therein and create a reservoir tie, to hold the reservoir onto the blade more securely. In certain embodiments, including highly soluble and friable lyophilised formulations, it is preferred that the friable reservoir may be entirely held within such holes thereby protected from breakage during puncture of the skin.

In an alternative embodiment, the microneedles, support members or reservoir medium (or portions thereof) may be physically separable from the patch or backing plate prior to DNA release. For example, in the embodiment where the skin piercing microneedles (or at least the tips thereof) are formed from the reservoir itself, after penetration of the skin the piercing protrusions separates from the base support thus allowing the patch to be removed from the skin, whilst leaving the reservoir behind in the skin. The separation of the reservoir from the backing plate may be by physical shearing or by biodegradation of part of the needles adjacent the backing plate.

One embodiment of this may be to cast the microprotrusion tips out of a relatively poorly soluble disaccharide reservoir medium (containing a dispersion of the agent to be delivered) followed by casting the remaining portion of the microprotrusion and backing plate out of a relatively easily soluble material. Once inserted into the skin, the relatively easily soluble microprotrusion shaft would degrade away, thereby allowing the patch to be removed from the skin, whilst leaving the tips within the skin. The tips, remaining in the skin can then slowly release the agent by slower biodegradation.

In another related embodiment of the present invention the devices may be electroporation devices. For example US 6261281 describes liquid intramuscular DNA vaccination followed by insertion of electrodes to pass an electric current across the muscle cells to enhance uptake of the DNA into the cells. WO 00/44438 describes needle patches coated with DNA in the absence of a reservoir medium, the metal needles being used as electrodes. Accordingly there is provided a electroporation device comprising a plurality of skin piercing elements which comprise a support member coated with an amorphous solid reservoir medium containing the DNA pharmaceutical agent, and a stabilising agent that inhibits the degradative effects of free radicals. One preferred embodiment of this are the devices described in WO 00/44438 the needles of which are coated with an amorphous reservoir medium containing a DNA vaccine and a stabilising agent that inhibits the degradative effects of free radicals.

The polyol biodegradable agent reservoir may be any made from any medium that fulfils the function required for the present invention. The reservoir must be capable of adhering to the support member to a sufficient extent that the reservoir remains physically stable and attached during prolonged storage, and also remains substantially intact during the administration procedure when the coated microneedles pierce the stratum corneum. The reservoir must also be capable of holding or containing a suspension or solution of agent to be delivered in any dry or partially dry form, which is released into the skin during biodegradation of the reservoir medium.

Biodegradation of the medium in the sense of the present invention means that the reservoir medium changes state, such that changes from its non-releasing to its releasing states whereby the agent enters into the skin. The release of the active agent may involve one or more physical and/or chemical processes such as hydration, diffusion, phase transition, crystallisation, dissolution, enzymatic reaction and/or chemical reaction. Depending on the choice of reservoir medium, biodegradation can be induced by one or more of the following: water, body fluids, humidity, body temperature, enzymes, catalysts and/or reactants. The change of the reservoir medium may therefore be induced by hydration, and warming associated with the higher humidity and temperature of the skin. The reservoir medium may then degrade by dissolution and/or swelling and/or change phase (crystalline or amorphous), thereby disintegrating or merely increase the permeation of the medium.

Preferably the medium dissolves, and is metabolised or expelled or excreted from the body, but the reservoir may alternatively remain attached to the support member to be removed from the skin when the device is removed. Release of the agent by dissolution of the reservoir medium is preferred.

Preferably the solid reservoir medium is a polyol (such as those described in WO96/03978). Suitable polyol reservoir media include carbohydrates (such as sugars), polysaccharides, substituted polyols such as hydrophobically derivatised carbohydrates, amino acids, biodegradable polymers or co-polymers such as poly(hydroxy acid)s, polyahhydrides, poly(ortho)esters, polyurethanes, poly(butyric acid)s, poly(valeric acid)s, and poly(lactide-co-caprolactone)s, or polylactide co-glycolide.

The solid reservoir may be in an amorphous or crystalline state and may also be partially amorphous and partially crystalline. Most preferably, however, all or substantially all of the reservoir is in an amorphous state. More preferably still is that the amorphous reservoir is in the form of a glass (US 5,098,893). Most preferably the reservoir is a sugar glass.

Particularly preferred reservoir media are those that stabilise the agent to be delivered over the period of storage. For example, antigen or agent dissolved or dispersed in a polyol glass or simply dried in a polyol are storage stable over prolonged periods of time (US 5,098,893, US 6,071,428; WO 98/16205; WO 96/05809; WO 96/03978; US 4,891,319; US 5,621,094; WO 96/33744). Such polyols form the preferred set of reservoir media.

Preferred polyols include sugars, including mono, di, tri, or oligo saccharides and their corresponding sugar alcohols. Suitable sugars for use in the present invention are well known in the art and include, trehalose, glucose, sucrose, lactose, fructose, galactose, mannose, maltulose, iso-maltulose and lactulose, maltose, or dextrose and sugar alcohols of the aforementioned such as mannitol, lactitol and maltitol. Sucrose, Lactose, Raffinose and Trehalose are preferred. Most preferred are glucose, trehalose or sucrose.

Preferably the DNA and stabilising agent are in a solid solution within the amorphous, and preferably glassy reservoir medium.

It is preferred that the reservoir medium forms an amorphous glass upon drying. The glass reservoir may have any glass transition temperature, but preferably it has a glass transition temperature that both stabilises the pharmaceutical agent during storage and also facilitates rapid release of the agent after insertion of the reservoir into the skin. Accordingly, the glass transition temperature is greater than ambient storage temperatures, but may be is around body temperature (such as, but not limited to 35-50°C).

The preferred reservoir media used to coat the skin-piercing members of the devices are those that release the pharmaceutical agent over a short period of time. The preferred reservoir formulations release substantially all of the agent within 10 minutes, more preferably within 5 minutes, more preferably within 2 minutes, more preferably within 1 minute, and most preferably within 30 seconds of insertion into a 1% agarose gel. When the formulations are used to administer DNA to the skin it is preferred that sufficient DNA is released to give its biological effect within 24 hours of administration to the skin, preferably sufficient DNA is released within 8 hours and most preferably within 1 hour of administration to the skin. Such fast releasing reservoirs can be achieved, for example, by thin coatings of amorphous glass reservoirs, particularly fast dissolving/swelling glassy reservoirs having low glass transition temperatures. It will be clear to the man skilled in the art that a low glass transition temperature can be achieved by selecting the appropriate glass forming sugar, and/or increasing humidity and/or ionic strength of the glass. Additionally, increased speed of dissolution of glass reservoirs may also be achieved by warming the device before or during application to the skin, pre-hydrating the skin or by administering liquid at the same time as inserting the microneedles into the skin (such as injecting liquid through the bores of the dry reservoir coated microcannulas) or adding additional agents to the formulation in order to decrease the dissolution time.

The DNA component of the present invention may be linear or open circular or supercoiled plasmid DNA, but may in a related form of the present invention the DNA may be in the form of a live attenuated bacterial or viral vector.

Preferably the DNA is in the form of a supercoiled plasmid. One major advantage of the present invention for these formulations is the fact that the DNA is stabilised so that upon release, it largely remains in its supercoiled form, and preferably in its monomeric supercoiled form.

Plasmid DNA stability can be defined in a number of ways and can be a relative phenomenon determined by the conditions of storage such as pH, humidity and temperature. For storage in the presence of iron ions on the coated reservoir, preferably >50% of plasmid remains supercoiled, (ccc, covalently closed circular), upon storage for 3 months at 4°C. More preferably, under the storage conditions described, >60% of plasmid remains ccc and more preferably, under these storage conditions, >90% of plasmid remains ccc for 3 months at 4°C. For coating on to non - metal ion based needles or microneedles, the stability of plasmid DNA would be preferably >60% and more preferably 80% and most preferably >90% ccc after 3 months storage at 4°C. More preferably, under these storage conditions, >90% of plasmid remains ccc for 1 year at 4°C, and more preferably >90% of plasmid remains ccc for 2 years at 4°C. Most preferably the above DNA stability is achieved under these conditions over the same time periods at 25°C.

The DNA within a solid reservoir medium coated onto sewing needles is preferably stabilised in its supercoiled (ccc) form during accelerated stability studies, and most preferably the DNA is stabilised in its monomeric ccc form. An example of an acellarated stability study is where dry coated needles are maintained at 37°C for 4 weeks followed by analysis of the DNA structure over time. In this type of study (as described in Example 12), preferably greater than 50% of the DNA remains in its ccc form, more preferably greater than 60% remains in its ccc form, more preferably greater than 70% remains in its ccc form, more preferably greater than 80% remains in its ccc form and most preferably greater than 90% remains in its ccc form. Under these conditions, and preferred levels of ccc, it is also preferred that the ratio of monomeric:dimeric ccc DNA is about 1 (such as within the range of 0.8-1.2, or more preferably within the range of 0.9-1.1 and most preferably within the range of 0.95-1.5), or greater than 1.

Studies to determine plasmid stability are well known to those skilled in the art and are described in (Evans *et al*., *Supra*; WO 97/40839). These include techniques to measure and quantify the percentage of supercoiled, ccc, plasmid DNA either by agarose gel electrophoresis, anion exchange HPLC, (Ferreira, G. et al., 1999, Pharm. Pharmacol. Commun., 5, pp57-59), or capillary gel electrophoresis, (Schmidt et al., 1999, Anal. Biochem., 274, 235-240). The ratio of monomeric:dimeric ccc can be measured by image intensity analysis after agarose gel electrophoresis (in the absence of any intercalating agents) and EtBr staining, using commercially available software such as Labworks 4.0 running on a UVP Bioimaging system.

The reservoir mediums of the present invention contain a stabilising agent that inhibits the degradative effects of free radicals. Preferred stabilising agents include stabilising metal ion chelating agents, while preferred metal ion chelating agents include inositol hexaphosphate, tripolyphosphate, succinic and malic acid, ethylenediamine tetraacetic acid (EDTA), tris (hydroxymethyl) amino methane (TRIS), Desferal, diethylenetriaminepentaacetic acid (DTPA) and ethylenediamindihydroxyphenylacetic acid (EDDHA). Other preferred stabilising agents are non-reducing free radical scavengers, and preferably such as agents are ethanol, methionine or glutathione. Other suitable chelators and scavengers (and those which are not suitable) may be readily identified by the man skilled in the art by routine experimentation (as described in WO 97/40839).

The amounts of the components present may be determined by the man skilled in the art, but generally are in the range of 0.1-10mM for the metal ion chelators, Ethanol is present in an amount up to about 5% (v/v), methionine is present at about 0.1 to 100mM and Glutathione is present at about 0.1 to 10% (v/v).

Preferred combinations of stabilising agents are (a) Phosphate buffered ethanol solution in combination with methionine or EDTA, (b) Tris buffered EDTA in combination with methionine or ethanol (or combinations of methionine and ethanol).

Particularly preferred formulations which may be combined with the DNA and the polyols: sucrose or trehalose in demetalated water or Phosphate or Tris based buffers and then dried onto the devices of the present invention are:
A. 10mM methionine and 2.9% ethanol
B. 3.7% ethanol and 1mM EDTA
C. 100mM Tris, 1mM EDTA and 10mM methionine and 2.9% ethanol
D. 100mM Tris, 1mM EDTA and 10mM methionine
E. 100mM Tris, 1mM EDTA and 2.9% ethanol

The preferred solid reservoir media in the devices of the present invention contain a metal ion chelating agent or a non-reducing free radical scavenger. Most preferably the solid reservoir media in the devices of the present invention contain both a metal ion chelating agent and a non-reducing free radical scavenger.

In addition to these stabilising agents, further steps may be taken to enhance the stability of the DNA in the solid vaccines. For example, the formulations may be made using solutions which themselves were demetalated before use (for example by using commercially available demetalating resin such as Chelex 100 from Biorad) and/or the formulation may be finalised in a high pH (such as pH 8-10)

The formulations comprising the agent to be delivered and biodegradable reservoir medium are preferably mixed in aqueous solution and then dried onto the support member or the formulation could be melted and then applied to the support member. A preferred process for coating the support members comprises making an aqueous solution of vaccine antigen and water soluble polyol (such as trehalose), followed by coating the solution onto the support members by dipping the member into the solution one or more times followed by drying at ambient temperature or lyophilisation to give a porous coating. In this process it is preferred that the initial solution of water soluble polyol or sugar is viscous, such as the viscosity achieved from 40% sugar.

Alternatively, minute picolitre volumes of solution or melted formulation may be sprayed onto individual support members by technology commonly used in the art of bubble-jet printers, followed by drying. An alternative method would be to prepare microspheres or microparticles or powders of amorphous formulation containing polyol such as sugar, using techniques known in the art (such as spray drying or spray freeze drying or drying and grinding) and by controlling the moisture content to achieve a relatively low glass transition temperature (for example 30°C), followed by spraying or dipping to bring the micropheres or microparticles or powders into contact with the support member heated to a temperature above that of the glass transition temperature of the microsphere (for example 45°C). The coated particles would then melt and adhere to the support member and then dry or the coated support member would be further dried (to remove residual moisture content) thereby increasing the glass transition temperature of the reservoir medium suitable for storage.

Alternatively, the support member may be coated using a freeze coating technique. For example, the temperature of the microneedle support member may be lowered below that of the freezing point of water (for example by dipping in liquid nitrogen) and then aqueous solutions of the reservoir medium and agent may be sprayed onto the cold support members, or the support members may be dipped into the solution of agent. In this way the agent and reservoir medium rapidly adheres to the microneedle support member, which can then be sublimed by lyophilisation, or evaporated at higher temperatures, to dry the reservoir coating.

Another method to coat the microneedle support is to dip them in a solvent, such as water (optionally comprising a surfactant to ensure good contact) then dipping wetted support members in a powdered form of the reservoir medium which is soluble in the solvent, followed by drying to remove the solvent.

In a preferred embodiment of the invention there is provided a process for coating a support member with a viscous solution of reservoir forming medium which is sufficiently fluid to allow sterile filtration through a 220 nm pore membrane. Accordingly there is provided a vaccine formulation comprising antigen in a filterable viscous sugar solution formulation. Preferred examples of such filterable viscous sugar solutions are solutions of between about 20 to about 50 % sugar (weight/volume of the final vaccine formulation prior to drying). More preferably the viscous filterable sugar solutions are in the range of about 30% to about 45% sugar, and most preferable are about 40% (weight sugar/volume of the final vaccine formulation prior to drying). In this context the most preferred sugar solutions comprise sucrose, raffinose, trehalose, glucose or lactose.

High concentration sugar solutions (e.g. 20-40%) are preferred as this increases the stability of the DNA in its monomeric ccc form.

Using these techniques each skin piercing microneedle may be loaded with relatively high amounts of pharmaceutical agent. Each piercing member preferably being loaded with up to 500 ng of DNA pharmaceutical, more preferably up to 1 µg of pharmaceutical DNA, more preferably up to 5µg of pharmaceutical DNA and most preferably up to 10µg of pharmaceutical DNA.

Preferably the vaccine formulations of the present invention contain DNA that encode an antigen or antigenic composition capable of eliciting an immune response against a human pathogen, which antigen or antigenic composition is derived from HIV-1, (such as tat, nef, gp120 or gp160), human herpes viruses, such as gD or derivatives thereof or Immediate Early protein such as ICP27 from HSV1 or HSV2, cytomegalovirus ((esp Human)(such as gB or derivatives thereof), Rotavirus (including live-attenuated viruses), Epstein Barr virus (such as gp350 or derivatives thereof), Varicella Zoster Virus (such as gpI, II and IE63), or from a hepatitis virus such as hepatitis B virus (for example Hepatitis B Surface antigen or a derivative thereof), hepatitis A virus, hepatitis C virus and hepatitis E virus, or from other viral pathogens, such as paramyxoviruses: Respiratory Syncytial virus (such as F and G proteins or derivatives thereof), parainfluenza virus, measles virus, mumps virus, human papilloma viruses (for example HPV6, 11, 16, 18, ..), flaviviruses (e.g. Yellow Fever Virus, Dengue Virus, Tick-borne encephalitis virus, Japanese Encephalitis Virus) or Influenza virus (whole live or inactivated virus, split influenza virus, grown in eggs or MDCK cells, or Vero cells or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as *Neisseria spp,* including *N. gonorrhea* and *N. meningitidis* (for example capsular polysaccharides and conjugates thereof, transferrin-binding proteins, lactoferrin binding proteins, PilC, adhesins); *S. pyogenes* (for example M proteins or fragments thereof, C5A protease, lipoteichoic acids), *S. agalactiae, S. mutans; H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B*. *parapertussis and B. bronchiseptica; Mycobacterium spp., including M. tuberculosis* (for example ESAT6, Antigen 85A, -B or -C), *M. bovis, M. leprae, M. avium, M. paratuberculosis, M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E. coli,* enteropathogenic *E. coli* (for example shiga toxin-like toxin or derivatives thereof); *Vibrio spp,* including *V. cholera* (for example cholera toxin or derivatives thereof); *Shigella spp,* including *S*. *sonnei, S. dysenteriae. S. flexnerii; Yersinia spp,* including *Y*. enterocolitica (for example a Yop protein) , *Y. pestis, Y. pseudotuberculosis; Campylobacter spp,* including *C. jejuni* (for example toxins, adhesins and invasins) and *C*. *coli; Salmonella spp,* including *S. typhi, S. paratyphi, S. choleraesuis, S. enteritidis; Listeria spp.,* including *L. monocytogenes; Helicobacter spp,* including *H.* pylori (for example urease, catalase, vacuolating toxin); *Pseudomonas spp,* including *P. aeruginosa; Staphylococcus spp.,* including *S. aureus, S. epidermidis; Enterococcus spp.,* including *E. faecalis, E. faecium; Clostridium spp.,* including *C.* tetani (for example tetanus toxin and derivative thereof), *C. botulinum* (for example botulinum toxin and derivative thereof), *C*. *difficile* (for example clostridium toxins A or B and derivatives thereof); *Bacillus spp.,* including *B. anthracis* (for example botulinum toxin and derivatives thereof); *Corynebacterium spp.,* including *C. diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii; Ehrlichia spp*., including *E. equi* and the agent of the Human Granulocytic Ehrlichiosis; *Rickettsia spp,* including *R. rickettsii; Chlamydia spp., including C. trachomatis* (for example MOMP, heparin-binding proteins), C. *pneumoniae* (for example MOMP, heparin-binding proteins), *C. psittaci; Leptospira spp.,* including *L. interrogans; Treponema spp.,* including *T. pallidum* (for example the rare outer membrane proteins*), T. denticola, T. hyodysenteriae;* or derived from parasites such as *Plasmodium spp.,* including *P. falciparum; Toxoplasma spp.,* including *T. gondii (for example SAG2, SAG3, Tg34); Entamoeba spp.,* including *E. histolytica; Babesia spp.,* including *B. microti; Trypanosoma spp.,* including *T. cruzi; Giardia spp.,* including *G. lamblia; Leshmania spp.,* including *L. major; Pneumocystis spp.,* including *P. carinii; Trichomonas spp.,* including *T. vaginalis; Schisostoma spp.,* including *S*. *mansoni,* or derived from yeast such as *Candida spp.,* including *C. albicans; Cryptococcus spp.,* including *C. neoformans.* Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464).

The DNA vaccine coated devices may be used for prophylactic or therapeutic vaccination and for priming and/or boosting the immune response.

One of the advantages of the present invention is the ability to co-formulate the DNA agent together with additional active agents, an ability that has been limited with other solid DNA pharmaceutical agents. For example, the DNA vaccine may further comprise an agent to enhance uptake of the DNA into the cell, an adjuvant or other immunostimulant to improve and/or direct the immune response, and may also further comprise pharmaceutically acceptable excipient(s).

For example, the solid pharmaceutical reservoir medium may preferably contain a DNA condensing agent for example spermidine or PEI (polyethyleneimine). Other excipients which may be included in the formulation include buffers, amino acids, phase change inhibitors ('crystal poisoners') which may be added to prevent phase change of the coating during processing or storage or inhibitors to prevent deleterious chemical reactions during processing or storage such Maillard reaction inhibitors like amino acids.

A preferred additional agent to the co-entrapped within the reservoir medium with the DNA is a DNAase inhibitor. One example of a DNAase inhibitor which is preferred is aurinticarboxylic acid (ATA, Glasspool-Malone, J. *et al*., (2000), Molecular Therapy **2**: 140-146).

In addition, the DNA vaccines of the present invention, may advantageously also include an adjuvant. Suitable adjuvants for vaccines of the present invention comprise those adjuvants that are capable of enhancing the antibody responses against the IgE peptide immunogen. Adjuvants are well known in the art (Vaccine Design - The Subunit and Adjuvant Approach, 1995, Pharmaceutical Biotechnology, Volume 6, Eds. Powell, M.F., and Newman, M.J., Plenum Press, New York and London, ISBN 0-306-44867-X). Suitable adjuvants for vaccines of the present invention comprise those adjuvants that are capable of enhancing the antibody responses against the immunogen. Suitable immunostimulatory agents include, but this list is by no means exhaustive and does not preclude other agents: synthetic imidazoquinolines such as imiquimod [S-26308, R-837], (Dockrell and Kinghorn, 2001, Journal of Antimicrobial Chemotherapy, 48, 751-755; Harrison, et al. 'Reduction of recurrent HSV disease using imiquimod alone or combined with a glycoprotein vaccine', Vaccine 19: 1820-1826, (2001)); and resiquimod [S-28463, R-848] (Vasilakos, et al. Adjuvant activites of immune response modifier R-848: Comparison with CpG ODN', Cellular immunology 204: 64-74 (2000).), Schiff bases of carbonyls and amines that are constitutively expressed on antigen presenting cell and T-cell surfaces, such as tucaresol (Rhodes, J. et al. Therapeutic potentiation of the immune system by costimulatory Schiff-base-forming drugs', Nature 377: 71-75 (1995)), cytokine, chemokine and co-stimulatory molecules as either protein or peptide, this would include pro-inflammatory cytokines such as GM-CSF, IL-1 alpha, IL-1 beta, TGF-alpha and TGF - beta, Th1 inducers such as interferon gamma, IL-2, IL-12, IL-15 and IL-18, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD40L, , other immunostimulatory targeting ligands such as CTLA-4 and L-selectin, apoptosis stimulating proteins and peptides such as Fas, (49), synthetic lipid based adjuvants, such as vaxfectin, (Reyes et al., 'Vaxfectin enhances antigen specific antibody titres and maintains Th1 type immune responses to plasmid DNA immunization', Vaccine 19: 3778-3786) squalene, alpha- tocopherol, polysorbate 80, DOPC and cholesterol, endotoxin, [LPS], Beutler, B., 'Endotoxin, 'Toll-like receptor 4, and the afferent limb of innate immunity', Current Opinion in Microbiology 3: 23-30 (2000)) ; CpG oligo- and di-nucleotides, Sato, Y. et al., 'Immunostimulatory DNA sequences necessary for effective intradermal gene immunization', Science 273 (5273): 352-354 (1996). Hemmi, H. et al., 'A Toll-like receptor recognizes bacterial DNA', Nature 408: 740-745, (2000) and other potential ligands that trigger Toll-like receptors to produce Th1-inducing cytokines, such as synthetic Mycobacterial lipoproteins, Mycobacterial protein p19, peptidoglycan, teichoic acid and lipid A.

Certain preferred adjuvants for eliciting a predominantly Thl-type response include, for example, a Lipid A derivative such as monophosphoryl lipid A, or preferably 3-de-O-acylated monophosphoryl lipid A. MPL^{®} adjuvants are available from Corixa Corporation (Seattle, WA; *see,* for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins.

In this aspect of the present invention the preferred immunostimulatory agent or adjuvant is immiquimod or other related molecules (such as resiquimod) as described in PCT patent application publication number WO 94/17043 (the contents of which are incorporated herein by reference).

In an embodiment of the invention, a polynucleotide is administered/delivered as "naked" DNA, for example as described in Ulmer et al., Science 259:1745-1749, 1993 and reviewed by Cohen, Science 259:1691-1692, 1993. The uptake of naked DNA may be increased by coating the DNA onto small microbeads beads, such as gold, or biodegradable beads, which are efficiently transported into the cells; or by using other well known transfection facilitating agents, such as Calcium Phosphate or DEAE dextran.

DNA may also be administered in conjunction with a carrier such as, for example, liposomes, and everything being entrapped in the reservoir medium. Typically such liposomes are cationic, for example imidazolium derivatives (WO95/14380), guanidine derivatives (WO95/14381), phosphatidyl choline derivatives (WO95/35301), piperazine derivatives (WO95/14651) and biguanide derivatives.

Examples of suitable pharmaceutically acceptable excipients include water, phosphate buffered saline, isotonic buffer solutions.

It is an intention of the present invention to administer agent or vaccine into the skin rapidly and with high yield of administration. This may be even further enhanced by a number of means, comprising the use of highly soluble carbohydrates as the reservoir medium, and also by agitating and/or heating the microneedle member during administration.

The amount of expressible DNA in each vaccine dose is selected as an amount which induces an immunoprotective response without significant adverse side effects in typical vaccinees. Such amount will vary depending upon which specific DNA construct is employed, however, it is expected that each dose will generally comprise 1-1000 µg of DNA, preferably 1-500 µg, more preferably 1-100 µg, of which 1 to 50µg is the most preferable range. An optimal amount for a particular vaccine can be ascertained by standard studies involving observation of appropriate immune responses in subjects. Following an initial vaccination, subjects may receive one or several booster immunisations adequately spaced.

The formulations of the present invention may be used for both prophylactic and therapeutic purposes. Accordingly, the present invention provides for a method of treating a mammal susceptible to or suffering from an infectious disease or cancer, or allergy, or autoimmune disease. In a further aspect of the present invention there is provided a vaccine as herein described for use in medicine. Vaccine preparation is generally described in New Trends and Developments in Vaccines, edited by Voller et al., University Park Press, Baltimore, Maryland, U.S.A. 1978.

The present invention is exemplified by, but not limited to, the following examples.

### Example 1, Demonstration of coating of needles with lyophilised plasmid DNA.

### 1.1 Plasmid preparation and formulations.

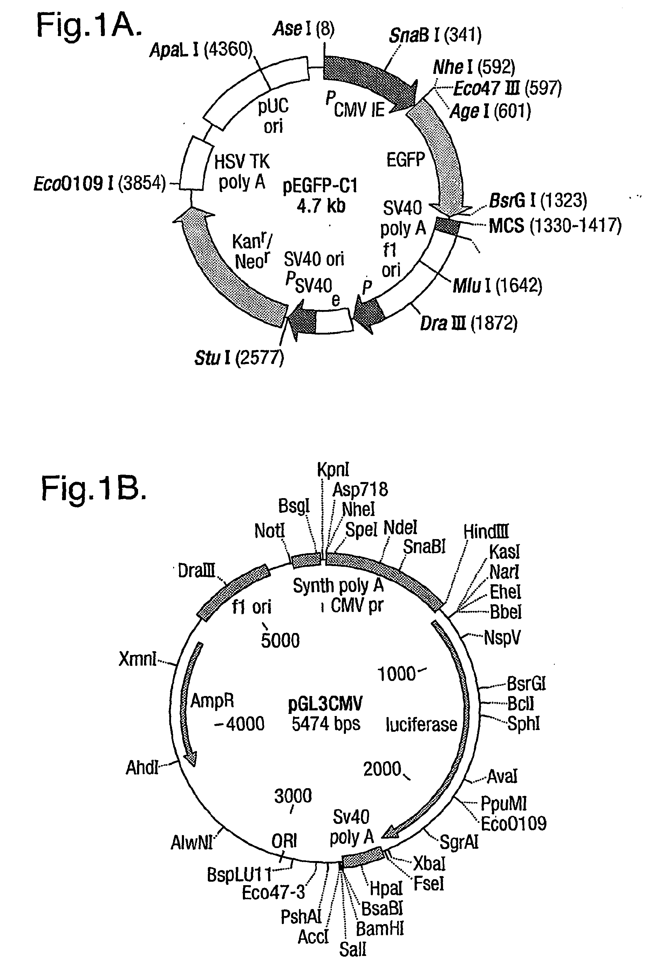

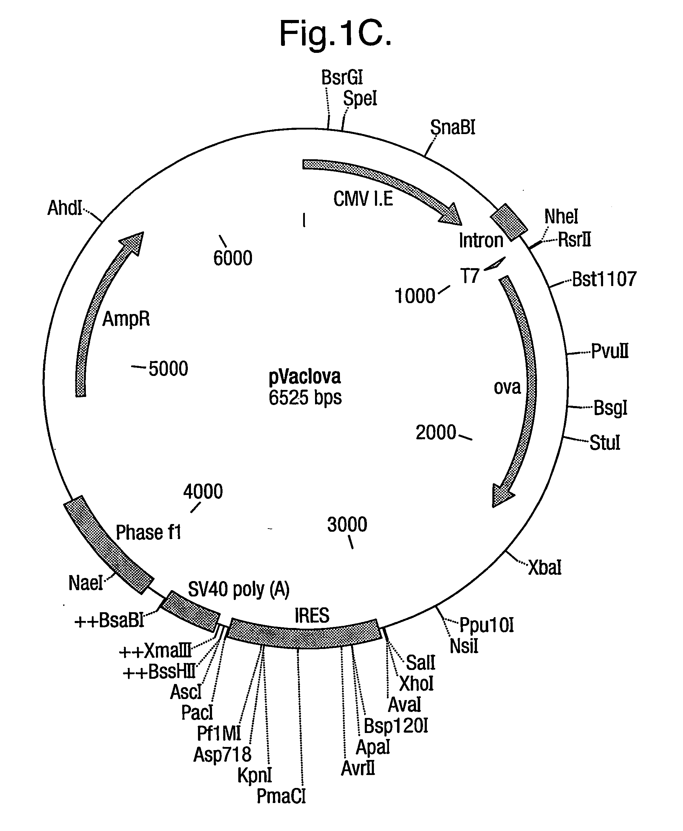

The plasmids used in this study are all shown in Figure 1. pEGFP-C1 is a GFP expression vector, (Clontech, Palo Alto, California, USA). pGL3CMV is a luciferase expression vector based upon pGL3 Basic, (Promega Corporation., Madison, Wisconsin, USA), where the CMV immediate early promoter drives luciferase expression.
pVAC1.ova is a chicken ovalbumin expression plasmid, constructed by ligating PCR amplified cDNA encoding chicken ovalbumin from pUGOVA, into the expression vector pVAC1. pVAC1 is a modification of the mammalian expression vector, pCI, (Promega), where the multiple cloning site, from EcoRI to Bst ZI, has been replaced by the EMCV IRES sequence flanked 5' by unique Nhe I, Rsr II and Xho I and 3' by unique Pac I, Asc I and Not I restriction enzyme sites, amplified from pGL3Basic, (Promega). Supercoiled plasmid DNA, (low endotoxin), was purified on a large scale, aproximately 100mg yield, to high purity using a combination of alkaline SDS lysis, ultrafiltration and anion exchange column chromatography.
Plasmids were resuspended in TE, (10mM TrisHCl, 1mM EDTA), pH 8.0 at 1ug / ul. And determined as >95% supercoiled upon analysis by agarose gel electrophoresis.

Plasmids were formulated in a variety of solutions, for coating needles, by a standard large-scale ethanol precipitation procedure. The precipitated DNA was resuspended directly into the aqueous formulation solutions at concentrations of 0.5 to 12 ug/ul, (See Chapter 1, Molecular Cloning: A Laboratory Manual, Sambrook, J. et al., 2nd Edition, 1989, CSH laboratory Press, Cold Spring Harbor, New York, USA).

### 1.2 Needles and coating procedures.

Size 8 sewing needles, (A817, Miliners, Milward, Coats Crafts, UK), were obtained from the John Lewis partnership, PLC, (London, UK.). Hypodermic needles, Microlance 3, 30G, 26G, 25G and 21G were obtained from Becton and Dickinson, (New Jersey, USA).

Sewing needles were coated by dipping once for 30 to 60 seconds into 1.5 ml of plasmid formulation, needle suspended from the rubber stopper of a 2ml glass lyophilisation vial, such that 1.0 to 1.5cm of the sewing needle was coated. Hypodermic needles were similarly coated being manually held in place with a 1ml syringe to balance in 1.5ml plasmid formulations in 2ml plastic screw capped tubes, Sarstedt, (Numbrecht, Germany).

Acid treated needles were dipped for 5 seconds in concentrated hydrochloric acid, (HCl), followed by distilled water washing and air drying on paper towels. Needles were then stored in a sterile glass beaker and subject to a single wet autoclave cycle for 20 minutes at 15 psi. Needles were then dried in a fume hood and stored at room temperature.

### 1.3 Lyophilisation and elution of plasmid DNA.

Coated needles were lyophilised under vacuum for a minimum of one hour at -45°C or below using a Modulyo 4K Freezer Dryer, (Edwards, Crawley, UK). DNA coated, lyophilised needles were either used immediately or stored sealed at 4°C.

Plasmid DNA was eluted from coated needles by shaking for 30 minutes in 0.5ml of 100mM Tris /HCl pH 8.0, 1mM EDTA, 10mM methionine, 2.9% ethanol in 2ml plastic screw capped tubes, Sarstedt, at room temperature. Plasmid DNA was then recovered by standard ethanol precipitation, (Sambrook, J. *et al., supra*), and resuspended in 20 to 30 ul of 100mM Tris /HCl pH 8.0, 1mM EDTA, 10mM methionine, 2.9% ethanol for application on to agarose gels.

### 1.4 Agarose gel electrophoresis and agarose gel 'needle release assay'.

Plasmid DNA and that eluted from needles after coating was routinely run on either 0.8% or 1.2% agarose E-gels containing ethidium bromide according to manufactures instructions, Invitrogen Corporation, (Carlsbad, California, USA).

To monitor the level of release of plasmid DNA from coated needles directly into agarose gels, 2.5cm thick, 1% agarose, (Ultra Pure, Life Technologies, Paisley, Scotland, UK), gels were poured. Needles were inserted into the agarose just behind the well to allow for plasmid DNA release from the needle into the agarose. Gel electrophoresis was then performed without ethidium bromide or other DNA visualisation agents at 100V, 100mA for 1 to 2 hours, (see Chapter 6 in Sambrook, J. *et al., supra*). DNA was then visualised by staining with with SYBR Gold, according to manufacturer's instructions, (Molecular Probes, Eugene, Oregon, USA.). Gel analysis was performed using the Visionworks package on the UVP8000 gel analysis system, (UVP Life Sciences, Cambridge, UK.).

### 1.5 Coating of sewing needles with high concentrations of DNA in aqueous solution containing stabilising agents.

Initial experiments to investigate the feasibility of coating sharp surfaces with plasmid DNA from solution were performed on acid treated sewing needles. Size 8 sewing needles were dipped once for 2-3hrs in plasmid DNA solutions ranging from 0.5ug/ul to 5ug/ul prepared in either water or 0.85% saline. Needles were carefully removed and lyophilised and then stored at 4°C, sealed for 60 hrs before DNA was eluted, by vigorous shaking in 500ul TE for 30 min. DNA was concentrated by ethanol preciptation and analysed by gel electrophoresis. DNA, <100ng, partially degraded, was recovered from the 5ug/ul saline sample. No other samples showed sufficient DNA to be detected on an agarose gel, (data not shown). It was thought likely that the acid treatment procedure was generating a combination of ferrous and ferric metal ions that were adversely affecting short-term plasmid DNA stability, (Evans *et al. Supra;* WO 97/40839).

To improve the needle coating procedure and reduce DNA degradation plasmid was formulated in aqueous solution in the presence of a number of chemicals, (eg. chelators and free radical scavengers), shown to increase plasmid stability in the presence of metal ions, (Evans *et al. Supra;* WO 97/40839). Sewing needles were also cleaned with methanol and air-dried to reduce metal ion formation. Plasmid DNA solutions were prepared at very high concentration 5ug/ul or 10ug/ul in 100mM Tris, 1mM EDTA, 10mM methionine, 2.9% ethanol pH 8.0, (formulation 2). Size 8 sewing needles were coated with DNA by dipping in solution, overnight at 4°C, once and needles were carefully removed and lyophilised and then DNA was immediately eluted, concentrated and analysed by gel electrophoresis.

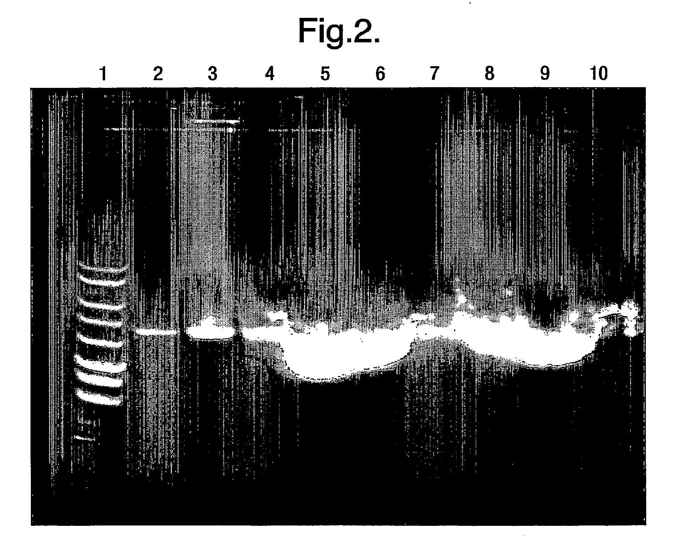

Data from such an analysis is shown in Figure 2, where coating of acid treated and methanol washed needles is compared.

The groups tested were run in lanes:
1) 1ug of 1kb DNA ladder, (Promega). 2) Eluted pEGFP-C1 plasmid from a 5ug/ul stock coated on to HCl treated sewing needles. 3) Eluted pEGFP-C1 plasmid from a 10ug/ul stock coated on to HCl treated sewing needles. 4) Eluted pEGFP-C1 plasmid from a 5ug/ul stock coated on to methanol washed sewing needles. 5) Eluted pEGFP-C1 plasmid from a 10ug/ul stock coated on to methanol washed sewing needles. 6) pEGFP-C1 plasmid standard - 0.5ug. 7) pEGFP-C1 plasmid standard - 1.0ug, 8) pEGFP-C1 plasmid standard - 5.0ug, 9) pEGFP-C1 plasmid standard -10.0 ug

In the absence of excess metal ions generated by acid treatment, non-degraded DNA, 1-2ug, was recovered from the 5ug/ul sample whereas the 10ug/ul sample yielded ≥ 10ug. This established the principle that DNA could be coated and lyophilised on to sharp objects such as sewing needles and eluted largely intact.

### Example 2, Stability of high concentrations of plasmid after coating on to needles.

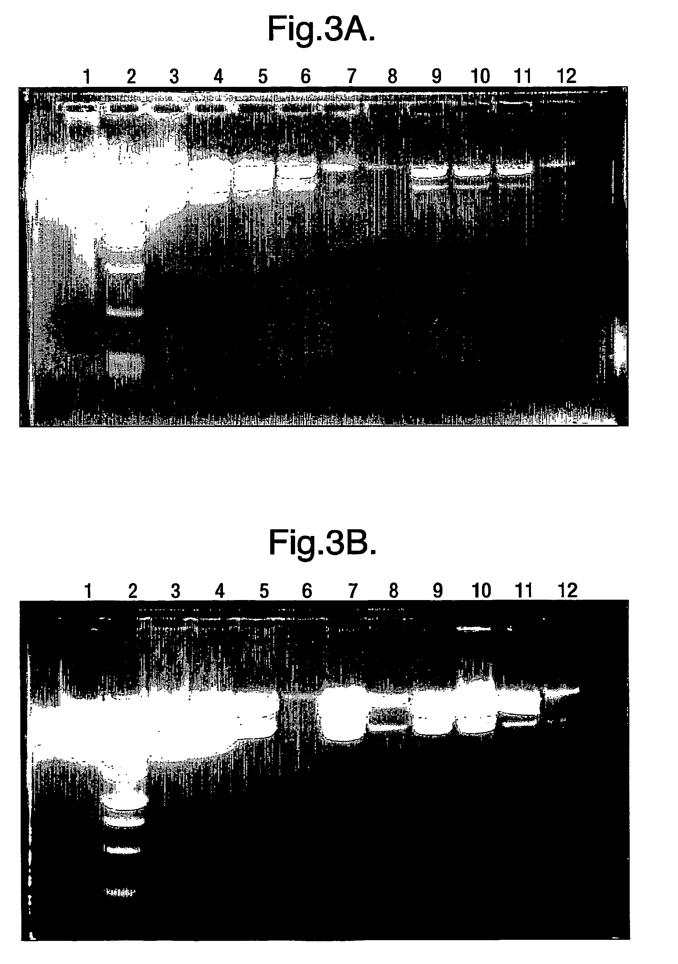

The reproducibility of needle coating and short-term stability of lyophilised plasmid DNA stored at 4°C on needles was then investigated. Plasmid DNA solutions of 5ug/ul or 10ug/ul in 100mM Tris, 1mM EDTA, 10mM methionine, 2.9% ethanol pH 8.0, (formulation 2), were coated onto size 8 sewing needles, lyophilised and the DNA was either immediately eluted or stored at 4°C for 1 - 4 days. Data for such an analysis is displayed in Figure 3, where duplicate needles are shown, stored for each time point:
**(A)** Eluted pEGFP-C1 plasmid from a 5uglul stock coated on to methanol washed sewing needles.
**(B)** Eluted pEGFP-C1 plasmid from a 10ug/ul stock coated on to methanol washed sewing needles.
In each gel the lanes represent: 1) pEGFP-C1 plasmid standard - 5.0ug **(A)**, 10.0ug **(B).** 2) 1ug of 1kb DNA ladder, (Promega). 3) & 4) Immediate elution after coating. 5) & 6) Elution after storage on sewing needles at 4°C for 16 hours. 7) & 8) Elution after storage on sewing needles at 4°C for 40 hours. 9) & 10) Elution after storage on sewing needles at 4°C for 64 hours. 11) & 12) Elution after storage on sewing needles at 4°C for 88 hours.

Large amounts of plasmid, up to 5ug, can be retained largely intact on coated, lyophilised needles and eluted after 66 to 88 hours storage at 4°C. However, coating, elution or storage was found to be somewhat variable, with the 10ug/ul sample showing the least variance. Conversion of covalently closed circular, (ccc), DNA, (bottom plasmid band, Fig. 3) to open circular, (oc), (top plasmid band, Fig. 3), seemed to be occurring over time upon storage. These issues were addressed with further improved formulations.

### Example 3, Improved short-term stability of high concentrations of plasmid after coating on to needles.

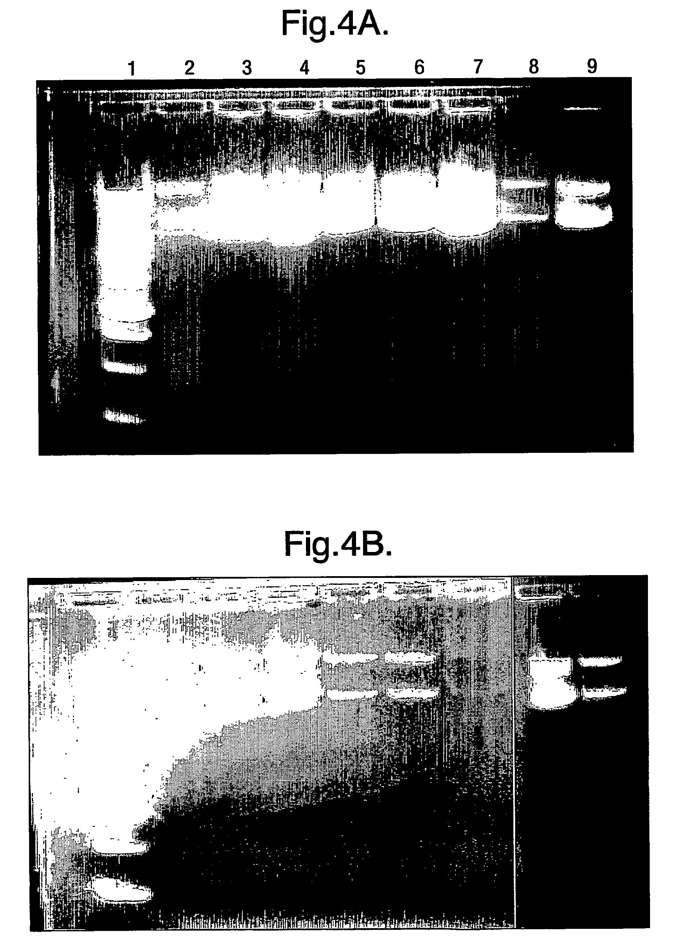

Further formulations of plasmid, in 100mM Tris, 1mM EDTA, 10mM methionine, 2.9% ethanol pH 8.0, (formulation 2), but with the additional presence of a low sugar (17.5%) "LS" or high sugar (40%) "HS" percentages of sugars, either trehalose or sucrose, were evaluated for needle coating and short-term stability after lyophilisation. Agarose gel electrophoresis of eluted DNA from such an analysis is shown in Figure 4. Formulations containing 17.5 % sucrose, (LS) or trehalose, (LT), (plasmid DNA at 5ug/ul), or 40% sucrose, (HS, plasmid DNA at 12ug/ul) or trehalose, (HT, plasmid DNA at 5ug/ul), were compared for needle coating, elution and DNA integrity.
Fig. 4 shows:
**(A)** Eluted pGL3CMV plasmid from formulation (HS) or formulation (HT), coated on to methanol washed sewing needles.
**(B)** Eluted pGL3CMV plasmid from formulations (LS) or (LT), coated on to methanol washed sewing needles.
   With the lanes in each diagram illustrating: 1) 1ug of 1kb DNA ladder, (Promega). 2) Immediate elution after coating, HS: **(A),** LS: **(B).** 3) Elution after storage on sewing needles at 4°C for 16 hours, HS: **(A),** LS: **(B).** 4) Elution after storage on sewing needles at 4°C for 40 hours, HS: **(A),** LS: **(B).** 5) Elution after storage on sewing needles at 4°C for 64 hours, HS: **(A),** LS: **(B).** 6) Immediate elution after coating, HT: **(A),** LT: **(B).** 7) Elution after storage on sewing needles at 4°C for 16 hours, HT: **(A),** LT: **(B).** 8) Elution after storage on sewing needles at 4°C for 40 hours, HT: **(A),** LT: **(B).** 9) Elution after storage on sewing needles at 4°C for 64 hours, HT: **(A),** LT: **(B).**

The presence of low and high concentrations of sugars, the latter particularly, (see Fig 4a), improved reproducibility and stability of lyophilised DNA coated onto needles. There was no obvious increase in the proportion of open circular plasmid DNA upon storage for 3 days at 4°C, in the presence of sugars and plasmid DNA remained as largely ccc format.

### Example 4, Release into agarose gels of high concentrations of plasmid from coated needles.

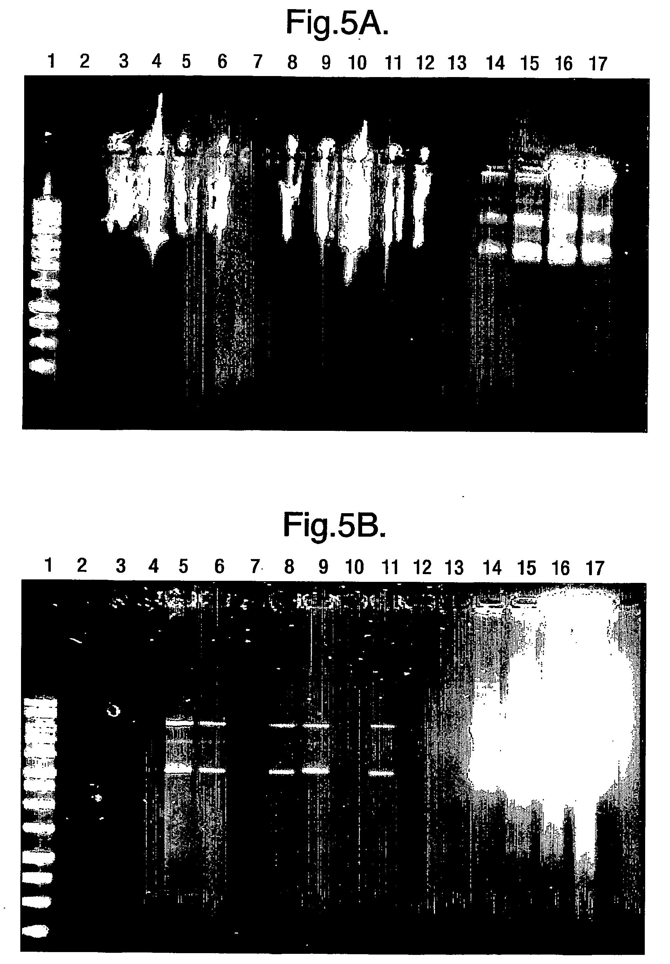

Experiments were performed to look at the rate of release or 'delivery' of lyophilised plasmid DNA, from the high sugar formulations, (HS, example 3), coated onto sewing needles, upon re-hydration. Stock plasmid DNA was resuspended in 100mM Tris /HCl pH 8.0, 1mM EDTA, 10mM methionine, 2.9% ethanol, (formulation 2), additionally containing 40% sucrose, (HS), at 12ug/ul or trehalose, (HT), at 5ug/ul for needle coating. DNA 'release' was achieved by placing the needles, freshly coated with lyophilised plasmid DNA, into thick, 1% agarose gels, just above the gel wells, for increasing increments of time from 15 sec. to 15 min. and performing electrophoresis. Gels were then stained with SYBR gold, the most sensitive DNA stain available, (see, Fig. 5A). Any remaining plasmid was eluted from the needles after 'stabbing' in to agarose and precipitated and analysed in SYBR gold stained agarose gels, as above, (see, Fig. 5B). The results, shown in Figure 5, demonstrated that the majority of the plasmid DNA, (>90%), about 10 ug, was released from the needles in 1 to 2 min.
Fig 5:
**(A)** Plasmid released from coated sewing needles after immediate insertion into an agarose gel for increasing time periods.
**(B)** Plasmid retained by and eluted from coated sewing needles after insertion into an agarose gel for increasing time periods, **(A).**
   with each lane representing:
   1) 1ug of 1kb DNA ladder, (Promega). 2) Gel release for 15 seconds, (HT). 3) Gel release for 60 seconds, (HT). 4) Gel release for 2 minutes, (HT). 5) Gel release for 5 minutes, (HT). 6) Gel release for 15 minutes, (HT). 7) Empty, 8) Gel release for 15 seconds, (HS). 9) Gel release for 60 seconds, (HS). 10) Gel release for 2 minutes, (HS). 11) Gel release for 5 minutes, (HS). 12) Gel release for 15 minutes, (HS).13) Empty. 14) pGL3CMV plasmid standard - 0.5ug. 15) pGL3CMV plasmid standard -1.0ug, 16) pGL3CMV plasmid standard - 5.0ug, 17) pGL3CMV plasmid standard- 12.0ug

### Example 5, Improved dose of plasmid coated onto a single hypodermic needle compared to a single sewing needle.

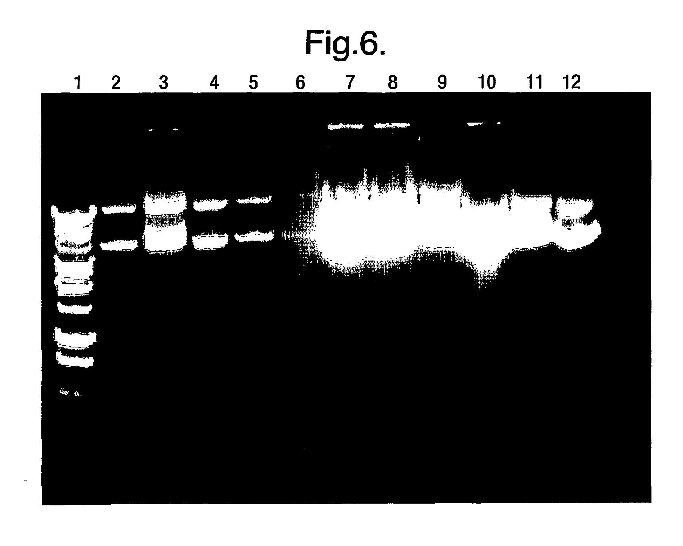

To increase the dose of plasmid DNA that could be coated and then lyophilized onto a single needle, it was hypothesized that a hollow hypodermic needle would present a greater surface area and accommodate more DNA than a solid sewing needle. 30G hypodermic needles were coated by the same plasmid DNA / needle coating procedure, (using the HS formulation, see Fig. 4b), alongside identical coating procedures for sewing needles and the amount of plasmid eluted was analysed by agarose gel electrophoresis. Five identical needles of each type were analysed. The results are shown in figure 6 for lanes1) 1ug of 1kb DNA ladder, (Promega), 2), 3), 4), 5) & 6) Eluted pGL3CMV plasmid from coated, methanol washed sewing needles, 7), 8), 9), 10 & 11) Eluted pGL3CMV plasmid from coated, 30G hypodermic needles. 12) pGL3CMV plasmid standard - 5.0ug.

The use of 30G hypodermic needles resulted in at least a two-fold increase in amount of plasmid coated over sewing needles. This suggests that substantially more DNA can be coated onto hollow needles compared to solid needles.

### Example 6, Optimal formulations for plasmid DNA release, after coating and lyophilization, from sewing and hypodermic needles.

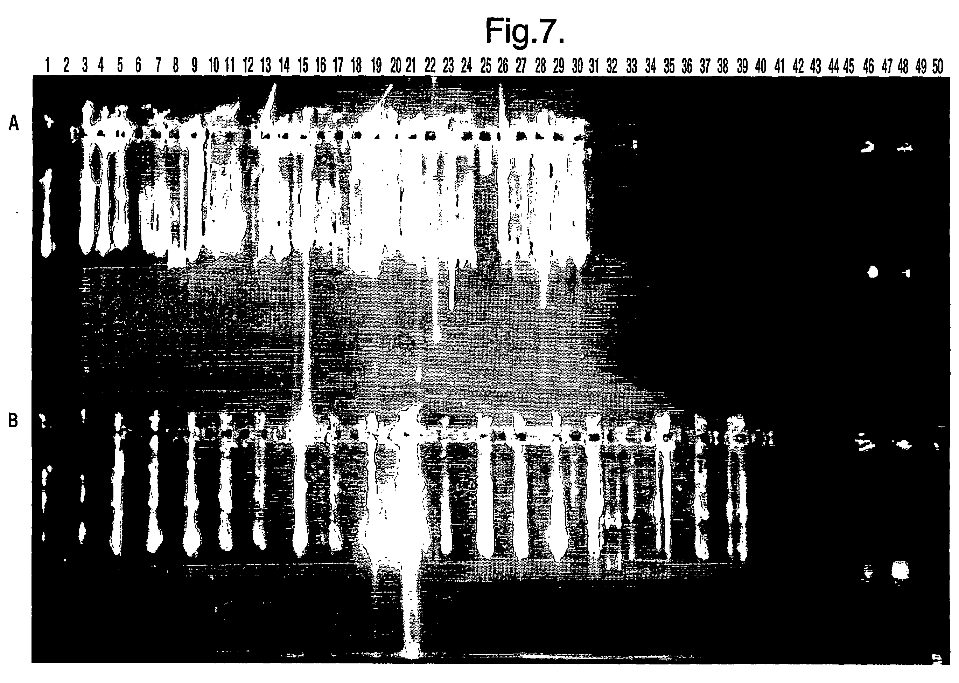

A series of different DNA formulations, lacking a number of individual components of the full HS formulation, (described in Example 3), were compared together with the full HS and LS formulations, for their ability to release DNA after coating and lyophilisation onto needles. A range of needles: size 10 sewing and hypodermics ranging through 21G, 25G, 26G to 30G were compared in this analysis. DNA release was assayed as described in Example 4, Fig. 5 by an 'agarose gel release assay', release time was approximately 2 minutes, followed by gel electrophoresis and such data is shown in Figure 7.
The lanes were pierced with needles coated with the following formulations:

| **FIG 7A** | **FIG 7B** |
|---|---|
| Formulation and needle combinations | |
| Lane:- | |
| 1) A1 | F1 |
| 2) A2 | Empty |
| 3) A3 | F2 |
| 4) A4 | Empty |
| 5) A5 | F3 |
| 6) Empty | Empty |
| 7) B1 | F4 |
| 8) B2 | Empty |
| 9) B3 | F5 |
| 10) B4 | Empty |
| 11) B5 | G1 |
| 12) Empty | Empty |
| 13) C1 | G2 |
| 14) C2 | Empty |
| 15) C3 | G3 |
| 16) C4 | Empty |
| 17) C5 | G4 |
| 18) Empty | Empty |
| 19) D1 | G5 |
| 20) D2 | Empty |
| 21) D3 | H1 |
| 22) D4 | Empty |
| 23) D5 | H2 |
| 24) Empty | Empty |
| 25) E1 | H3 |
| 26) E2 | Empty |
| 27) E3 | H4 |
| 28) E4 | Empty |
| 29) E5 | H5 |
| 30) Empty | Empty |
| 31) Empty | I1 |
| 32) Empty | Empty |
| 33) Empty | I2 |
| 34) Empty | Empty |
| 35) Empty | I3 |
| 36) Empty | Empty |
| 37) Empty | I4 |
| 38) Empty | Empty |
| 39) Empty | I5 |
| 40 - 45) Empty | Empty |
| 46) pVaclova plasmid standard - 0.5ug. | pVaclova plasmid standard - 0.5ug |
| 47) Empty | Empty |
| 48) pVaclova plasmid standard - 5.0ug | pVaclova plasmid standard - 5.0ug |
| 49) Empty | Empty |
| 50) 1ug of 1kb DNA ladder, (Promega). | 1ug of 1kb DNA ladder, (Promega). |

| | |
|---|---|
| With the formulations described using the following code: A) 40% sucrose, 100mM Tris /HCl pH 8.0, 1mM EDTA, 10mM methionine, 2.9% ethanol, (HS), B) 17.5% sucrose, 100mM Tris /HCl pH 8.0, 1mM EDTA, 10mM methionine, 2.9% ethanol, (LS), C) 40% sucrose, 100mM Tris /HCl pH 8.0, 1mM EDTA, 2.9% ethanol, (HS - methionine), D) 40% sucrose, 100mM Tris /HCl pH 8.0, 1mM EDTA, 10mM methionine, (HS - ethanol), E) 100mM Tris /HCl pH 8.0, 1mM EDTA, 10mM methionine, 2.9% ethanol, (formulation 2), F) 100mM Tris /HCl pH 8.0, 1mM EDTA, 2.9% ethanol, (formulation 2 - methionine), G) 100mM Tris /HCl pH 8.0, 1mM EDTA, 10mM methionine, (formulation 2 - ethanol), H) 40% sucrose in water, I) 10mM Tris/HCl pH 8.0, 1mM EDTA, 1 - Sewing needles, size 8, 2 - 30G hypodermic needles, 3 - 26G hypodermic needles, 4 - 25G hypodermic needles, 5 - 21G hypodermic needles. | |

The data suggests that the preferred formulations for optimal DNA release in this assay are those containing sucrose, (either 17.5 %, or 40%) or a full formulation of chelators and free-radical scavengers, (formulation 2) or more preferably a combination of both. The data also demonstrates that DNA release in this assay, for the majority of formulations tested, was best using the sewing needles and hypodermic needles of bore size greater than 26G, (26G optimal), with 30G hypodermic needles being poorest for DNA release in this assay.

### Example 7, Optimal formulations to stabilise plasmid DNA after coating, lyophilization and storage on needles.

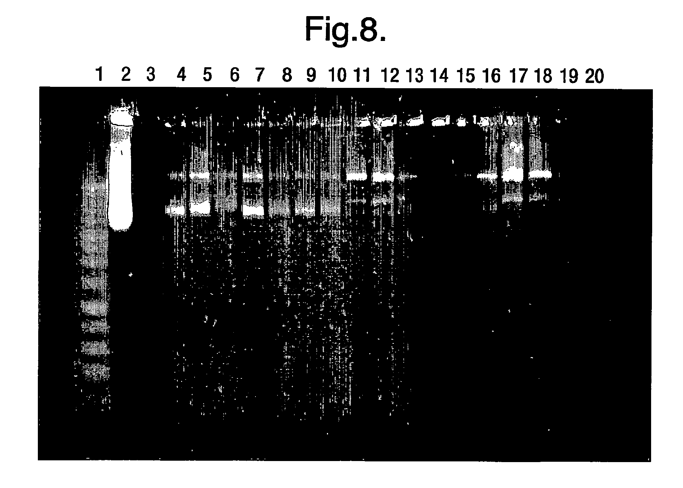

The series of different DNA formulations, lacking a number of individual components of the full HS formulation, (Example 3), as described in Example 6 and Fig. 7, were compared for their ability to stabilise supercoiled plasmid DNA, after coating and lyophilisation onto needles, upon short term storage. Sewing needles, methanol washed, were coated, lyophilised and stored at 4°C for 7 days. Plasmid DNA was then eluted and recovered in the standard manner and subject to agarose gel electrophoresis, (100V, 100mA for 2 hours), in the absence of intercalating agents, (Sambrook, J. *et al., supra*). The integrity of the eluted plasmid DNA was then monitored after staining with SYBR gold and visualisation under UV light. Such data is shown in Fig. 8, which shows plasmid DNA eluted from methanol-washed sewing needles after storage which were formulated in a variety of different formulations, A - I, as described in Example 6.
Lanes:-
1) 1ug of 1kb DNA ladder, (Promega).
2) pVaclova plasmid standard - 5.0ug
3) & (4) Formulation A, (sample lost- lane 3)
5) & (6) Formulation B
7) & (8) Formulation C
9) & (10) Formulation D
11) & (12) Formulation E
13) & (14) Formulation F
15) & (16) Formulation G
17) & (18) Formulation H
19) & (20) Formulation I

The data confirms that formulations containing a combination of sugar with chelating agents and free radical scavengers generate the greatest and most reproducible yield of plasmid DNA when eluted from needles. The data suggests that plasmid DNA is predominately maintained for release in the monomeric supercoiled form only in formulations that contain a combination of sugar with chelating agents and free radical scavengers.

### Example 8, Demonstration of in vivo delivery of functionally active plasmid DNA from coated, lyophilised needles.

### In vivo delivery and luciferase assays.

Plasmid DNA delivery from coated needles was performed into Balb/c x C3H F1 female mice both intramuscularly, (IM), and intradermally, (ID). For IM delivery a DNA coated 30G hypodermic needle was inserted into a pre-shaved femur muscle for 10 to 20 seconds before removal. For ID delivery mice were anaesthetised with isofluorane and a coated 30G hypodermic needle was inserted into a pre-shaved area of abdomen under low powered microscopy for 2 minutes. As positive controls, 10µg of plasmid DNA in saline was injected by standard procedure both IM and ID. Groups of 10 animals or tissues were analysed versus six positive controls. Mice were sacrificed and samples were removed 48 hours post plasmid delivery and snap frozen in liquid nitrogen.

Muscle or skin samples were thawed to room temperature and disrupted in 500µl of passive lysis buffer, (Promega Corporation, Madison, Wisconsin, USA), using an IKA Labortechnic Ultra turrax T8 polytron. Luciferase enzyme activity was determined using a luciferase assay kit, (Promega). 40µl of the lysate (in duplicate) was assayed together with 200µl of luciferase assay reagent (Promega) in a black 96 well plate, (Nunc). Luciferase activity (RLU) was measured as counts per second in the TopCountNXT HTS scintillation and luminescence counter, (Packard). Total protein concentration was calculated by Coomassie Plus protein assay reagent kit (Pierce) using the manufacturer's protocol. Briefly, 5µl of cell lysate was assayed together with 145µl of water (Sigma) and 150µl of coomasie blue reagent in 96 well flat-bottomed plates (Costar). The absorbance was measured at 595nm on a Molecular Devices Spectra Max 340. Luciferase activity was expressed as relative light units (RLU)/mg of total protein.

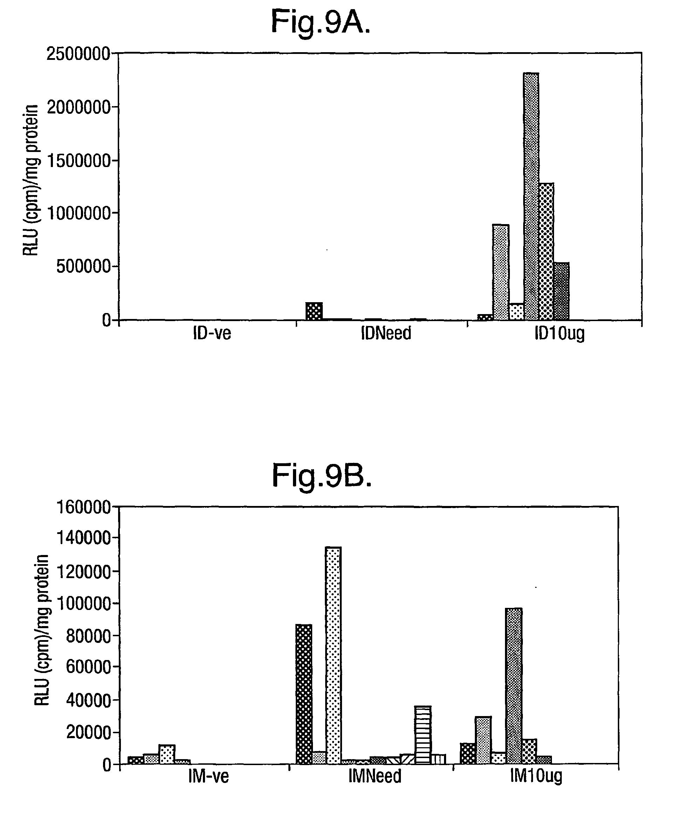

Data from such an experiment is shown in Figure 9, where luciferase activity derived from plasmid 'released from coated needles' is compared to that derived from plasmid delivered by standard IM, (Fig. 9A), and ID, (Fig. 9B), injections. Data suggested that at least 1/10 mice were positive for luciferase actvity after ID DNA release from coated needle administration and at least 3/10 mice were positive after the similar IM procedure. This demonstrates the principle that plasmid DNA can be released from these formulations when coated onto needles in a transcriptionally active form to allow expression of an encoded gene or antigen.

### Example 9, Demonstration that in vivo delivery of plasmid DNA, intradermally, (ID), from coated, lyophilised needles can show similar efficiency of gene expression to injection of liquid plasmid DNA.

### In vivo delivery and luciferase assays.

This was performed as described in Example 8 for ID delivery to mouse skin. As in example 8, all DNA formulations unless otherwise stated, used for gene delivery *in vivo,* contained pGL3CMV plasmid DNA at 10mg/ml and 40% sucrose, 100mM Tris /HCl pH 8.0, 1mM EDTA, 10mM methionine and 2.9% ethanol, and were coated onto sowing needles by lyophilisation as described previously.

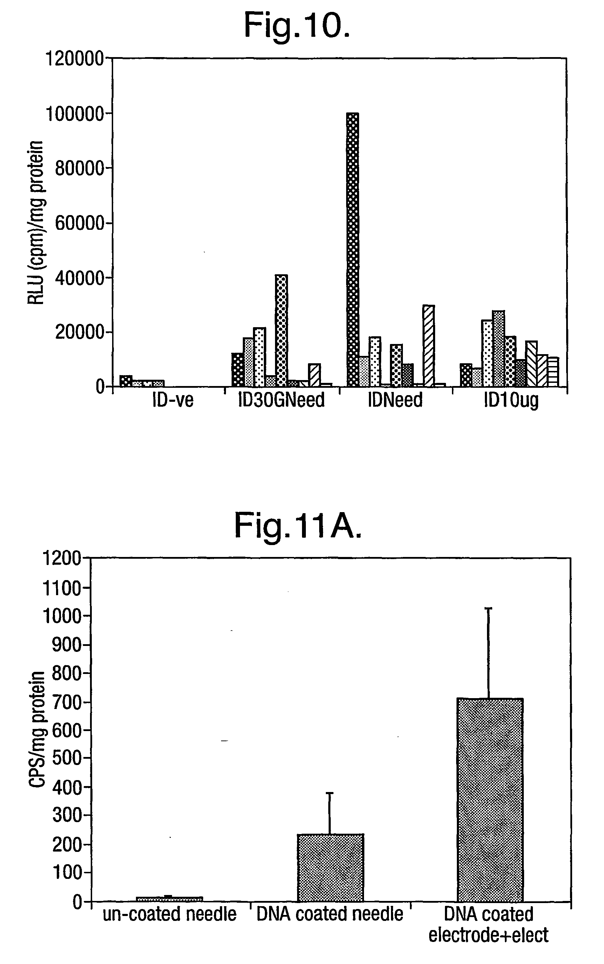

Data from an experiment, where luciferase activity derived from plasmid 'released from coated needles', (either 30G hypodermic or sowing needles), is compared to that derived from plasmid delivered by standard ID injection is shown in Figure 10. Data suggested that at least 5/9 mice were positive for luciferase actvity after ID DNA release from hypodermic needle injection and at least 6/9 mice were positive for luciferase actvity after ID DNA release from sowing needle injection. The levels of gene expression in individual animals are generally comparable to, or higher than, those animals to which 10µg of plasmid DNA had been delivered by ID injection.

### Example 10, Demonstration that in vivo delivery of functionally active plasmid DNA from coated, lyophilised needles can be enhanced by electroporation.

### In vivo delivery and luciferase assays.

This was performed as described in Example 8 for ID and IM delivery to mouse skin, except that for both ID and IM delivery, two sewing needles were inserted simultaneously to allow DNA release and needles remained in the tissue for 2 minutes in both cases.

Electroporation was performed after 2 minutes of DNA delivery, using a BTX 830 square wave electroporation device, (BTX, California, USA), using 5 mm separated 2 needle-array electrodes that had been similarly coated with plasmid DNA /sucrose formulations plus excipients.

For the ID delivery experiment shown in Figure 11A, the electroporation conditions were: 875 volts, 3 pulses of 100 microseconds followed by reverse polarity and 3 pulses of 100 microseconds with an interpulse delay of 125 milliseconds, (Glasspool-Malone, J. *et al.,* (2000), Molecular Therapy **2**: 140-146). The data is presented as the mean of 9 animals, 3 in the case of the uncoated needles, as luciferase activity in counts per second, (CPS) / mg protein. In this experiment all 9 animals where luciferase activity derived from plasmid, released from coated needles, were positive for gene expression as were all 9 animals where luciferase activity derived from plasmid, released from coated electrodes, and boosted with electroporation, (data not shown). The data suggests that electroporation boosts the level of gene expression from ID coated needle delivery an average of 2 to 3-fold under these conditions.

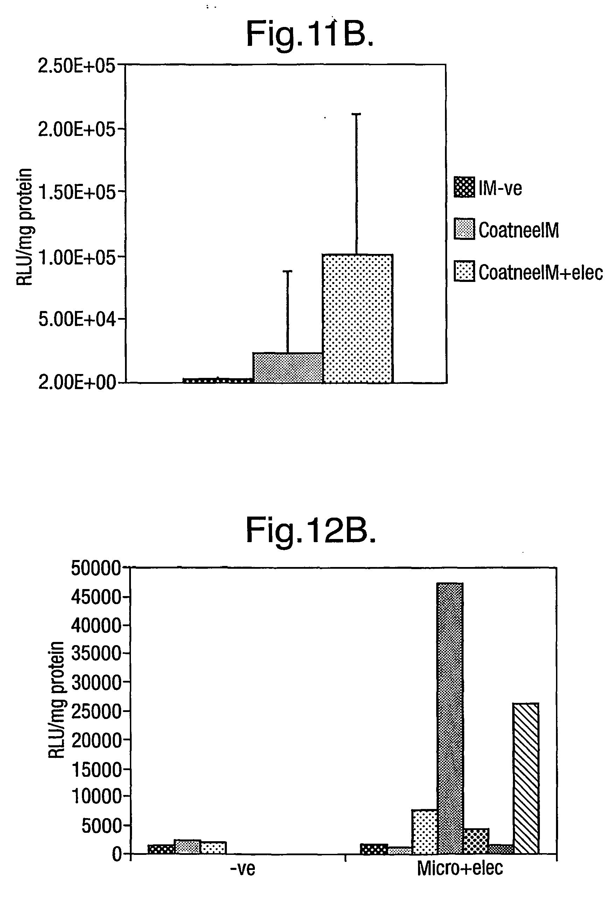

For the IM delivery experiment shown in Figure 11B, the electroporation conditions were: 900 volts, 3 pulses of 100 microseconds followed by reverse polarity and 3 pulses of 100 microseconds with an interpulse delay of 1 second, (Vicat, J., *et al.,* (2000), Human Gene Therapy **11**: 909-916). The data is presented as the mean of 9 animals, 3 in the case of the uncoated needles, as luciferase activity in relative light units, (RLU) / mg protein. In this experiment 1/9 animals where luciferase activity derived from plasmid, released from coated needles, were positive for gene expression whereas 6/9 animals where luciferase activity derived from plasmid, released from coated electrodes, and boosted with electroporation were positive for gene expression, (data not shown). The data suggests that electroporation boosts the level of gene expression from IM coated needle delivery an average of around 5 fold under these conditions. It also suggests that electroporation can increase the number of animals that are positive for gene expression derived from plasmid released from coated needles or electrodes.

### Example 11, Demonstration of in vivo delivery of functionally active plasmid DNA from coated, lyophilised microneedles and electroporation.

### In vivo delivery and luciferase assays.

This was performed as described in Example 8 for ID delivery to mouse skin, but with the following modifications. Cross-shaped hollow out-of-wafer-plane silicon microneedles with openings in the shaft were manufactured by a deep reactive ion etching, (DRIE), process, (Griss, P. & Stemme, G., 'Side-Opened Out-of-Plane Microneedles for Microfluidic Transdermal Liquid Transfer', (2002), In 'Micromachined Interfaces for Medical and Biochemical Applications', PHD Thesis, Griss, P., Royal Institute of Technology, Stockholm, Sweden). Microneedle parameters were for 5mm x 5mm square silicon microchips with an equidistant array of 100 microneedles, as 10 by 10. Individual microneedles were 240-250 microns in length. The 5mm x 5mm square silicon microchips were fixed centrally onto 1cm square holding plates to allow for application to mouse skin. The silicon microchips were coated by spreading 10µl of DNA / sugar / excipient formulation, onto the surface of the microneedle with a Gilson pipette, and lyophilising the coated microneedle, as described previously. These were placed on to the skin of pre-shaved Balb/c mice, at the lower back above the base of the tail. Mouse skin had been pre-hydrated in this region by the application of 5µl of phosphate buffered saline to the microneedle application site.

Mice were maintained under general anaesthesia using an oxygen-controlled inhaled Isoflourane mask and were given Rimadyl, (Carprofen), as an analgesic at a sub-cutaneous dose of 5mg/Kg, (delivered in 20µl/mouse), whilst under general anaesthesia but prior to microneedle application.

Electroporation was performed after 2 minutes of DNA delivery, using a BTX 830 square wave electroporation device, (BTX, California, USA), using a 1cm square calliper electrode separated by 1mm, on the fold of shaved skin where the microneedle had been applied. Parameters used were 75 volts, 3 pulses of 20 milliseconds with an interpulse delay of 100 milliseconds, (Zhang, L et al., (2002), Biochim. Biophysic. Acta 1572: 1-9).

Data from an experiment, where luciferase activity derived from plasmid 'released from coated microneedles' boosted with electroporation, (Micro + elec), is shown in FIG 12. In this experiment 4/7 animals were positive for gene expression and show luciferase activity derived from plasmid, released from coated microneedles, after electroporation and demonstrate that this can act as a gene delivery system to the skin.

### Example 12, Stability of plasmid DNA, in different sugar formulations, after coating, lyophilization and storage on needles, at 37°C.

A similar procedure was conducted to that described in Example 7 to compare the plasmid DNA stability of a series of different DNA formulations where either the amount of sucrose or the type of sugar used in the formulation was varied. All other excipients previously described as optimal for DNA stability and release remained present in all formulations, (ie. 100mM TrisHCl pH8.0, 1mM EDTA, 10mM methionine and 2.9% ethanol). The formulations were compared for their ability to stabilise supercoiled plasmid DNA, after coating and lyophilisation onto needles, upon storage for up to one month at 37°C, (accelerated DNA stability study). Plasmid DNA was then eluted and recovered in the standard manner and subject to agarose gel electrophoresis, (100V, 100mA for 2 hours), in the absence of intercalating agents, (Sambrook, J. *et al., supra*). The integrity of the eluted plasmid DNA was then monitored after staining with ethidium bromide and visualisation under UV light. The percentage of supercoiled monomeric and dimeric plasmid forms and also any linear and open circular forms from these samples were measured as image intensity using the Labworks 4.0 image analysis software on the UVP Bioimaging System.

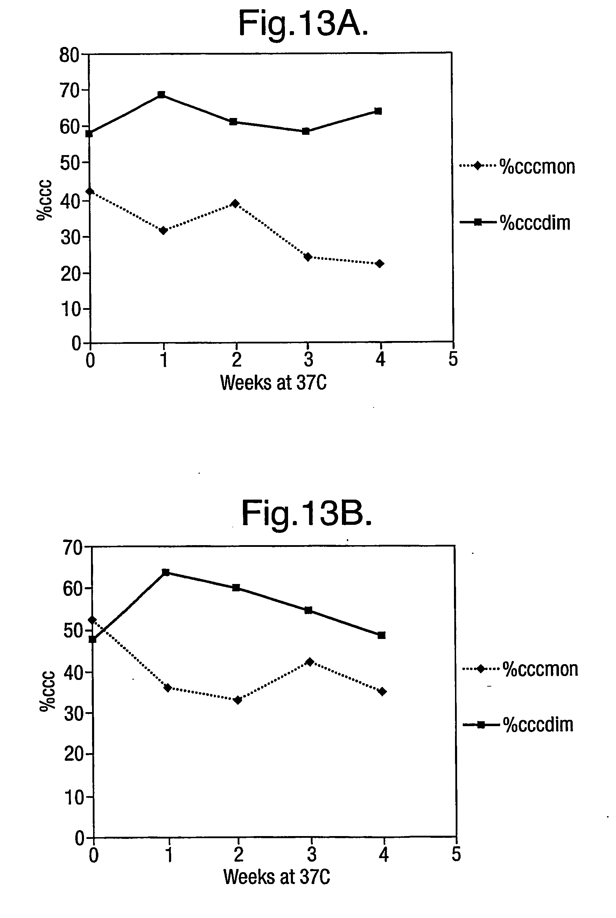

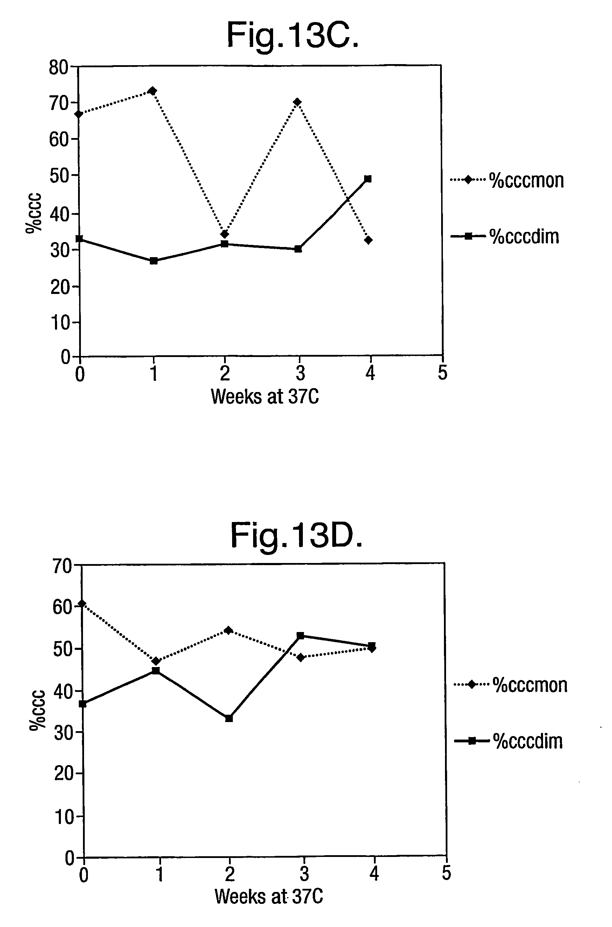

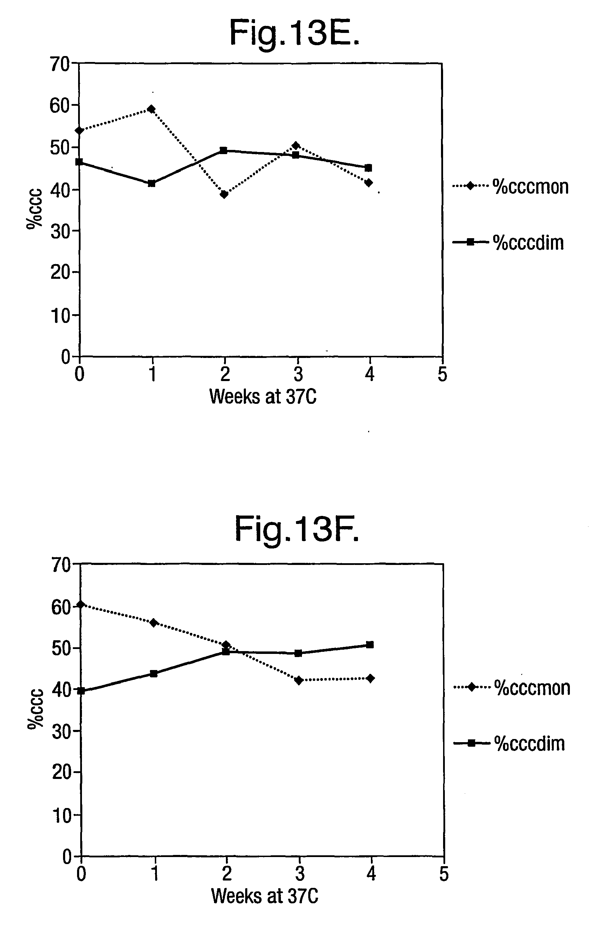

The data is displayed in FIG 13, as a graphical plot of the percentage of supercoiled plasmid, (%ccc), both monomeric, (%cccmon), and dimeric, (%cccdim), plasmid forms; after coating and lyophilization onto sowing needles and storage at 37°C. The plasmid formulations used contain varying amounts of sugars: FIG 13A: 5% sucrose, FIG 13B: 10% sucrose, FIG 13C: 17.5% sucrose, FIG 13D: 40% sucrose, FIG 13E: 40% trehalose, FIG 13F: 40% glucose.

The data suggest that all the formulations containing sugars maintain a high degree of plasmid stability, even after storage at 37°C for up to one month, greater than 80% and up to 98% of the plasmid remains in a supercoiled form. For formulations containing sugar levels of 40%, (w/v), the balance between the monomeric and dimeric plasmid forms remains relatively constant, with the preferred monomeric form predominating in sugar formulations varying from trehalose to sucrose to glucose, (FIGs 13D, 13E & 13F). For formulations containing lower concentrations of sucrose, the dimeric form tends to predominate over the monomer, especially upon prolonged storage at 37°C, (FIGs 13A, 13B & 13C). In general the data are consistant with the higher the level of sugar present in the formulation leading to greater stability of plasmid DNA.

### Example 13, Demonstration of amorphous glass formation after lyophilization of plasmid DNA, in sucrose formulations containing excipients.

### Analysis of lyophilised, plasmid DNA formulations by differential scanning calorimetry, (DSC).

Samples of lyophilised DNA / sucrose formulations were prepared containing plasmid DNA, (10mg/ml) in 40% sucrose and also lyophilised samples were prepared additionally containing 100mM TrisHCl pH8.0, 1mm EDTA, 10mM methionine and 2.9% ethanol. Samples were split and subject to either 1 hour or 24 hour lyophilization cycles. The samples were then subject to analysis by differential scanning calorimetry, (DSC), to determine the solid state form. This was performed on a TA Instruments DSC2920 machine over a temperature range from 25°C to 300°C, using nitrogen as the purge gas with a flow rate of 20ml / min. The sample pan type was pinhole aluminium and the sample weight was determined on the day of analysis on a Mettler M3 balance.

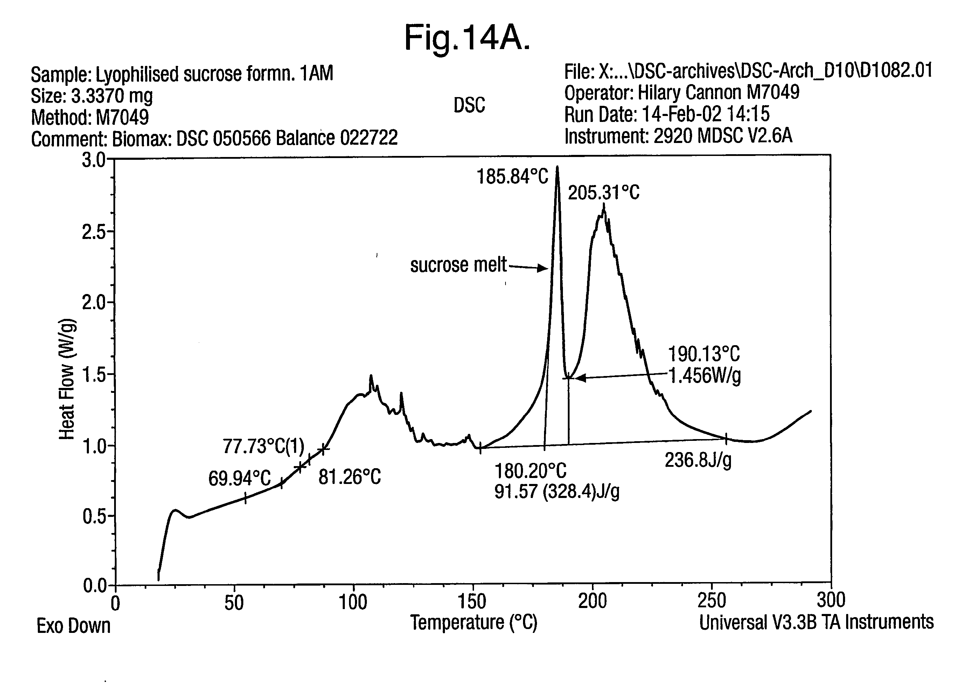

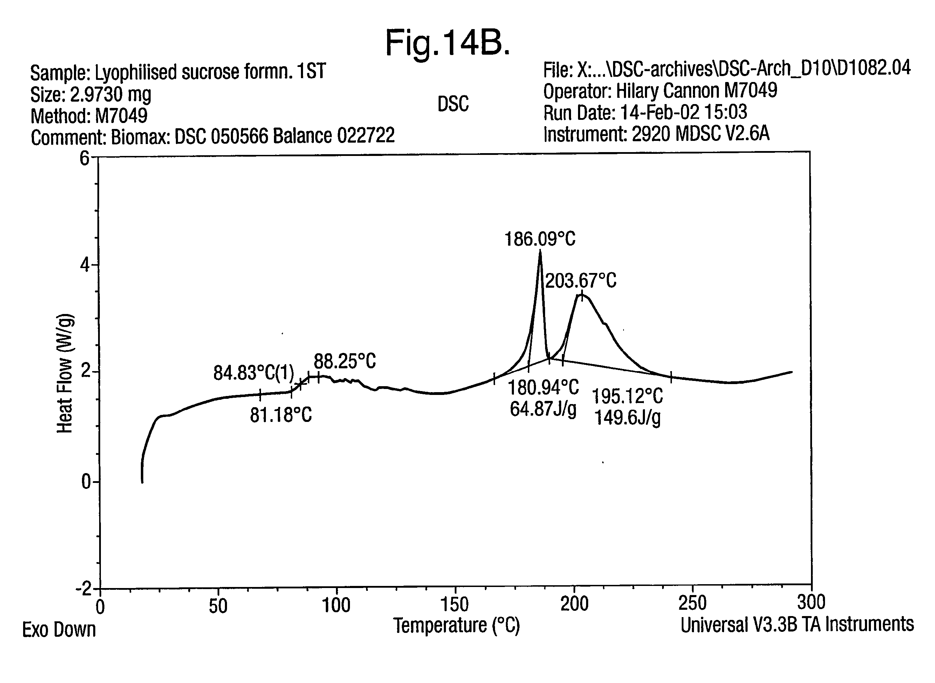

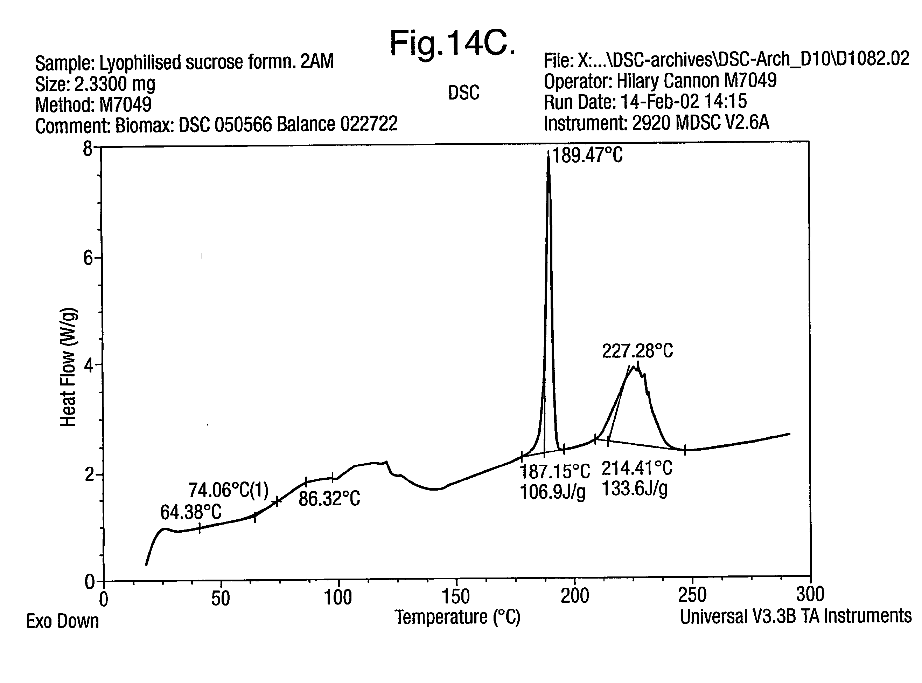

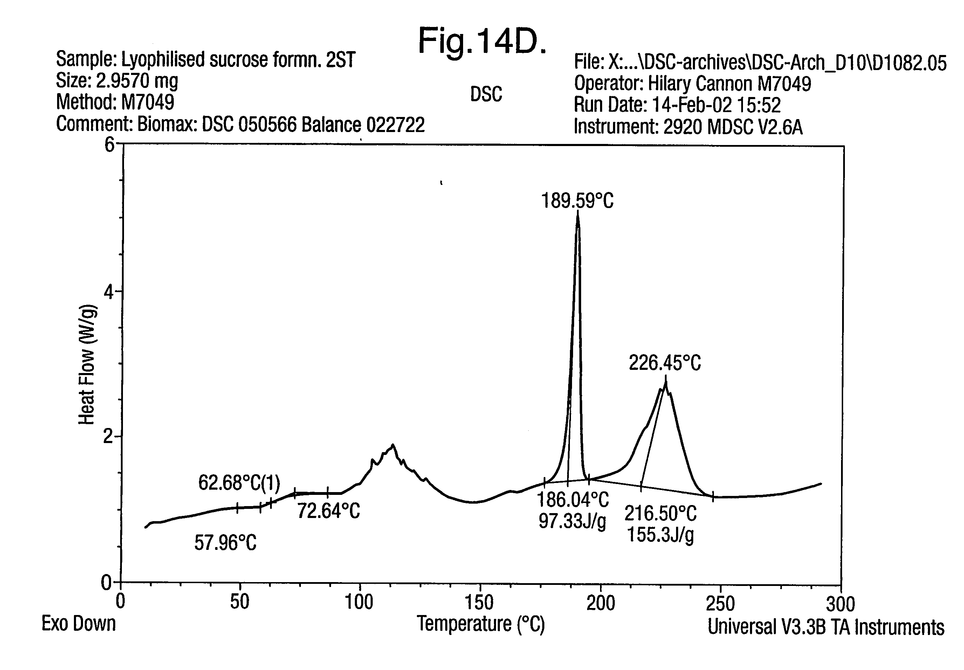

The data is displayed in FIG 14. All samples contain plasmid DNA, (10mglml), in 40% sucrose. Fig 14A & B: formulations also contain: 100mM TrisHCl pH8.0, 1mM EDTA, 10mM methionine and 2.9% ethanol; Fig 14A & C represent a 24 hour lyophilization cycle; Fig 14B & D represent a 1 hour lyophilization cycle. The data suggest that all the samples contain amorphous sucrose with sucrose glass transition temperatures being observed at about 78°C, (Fig 14A), 85°C, (Fig14B), 74°C, (Fig14C) and 63°C, (Fig14D), which fit well with published values in the literature, (5). The data suggests that both short and long lyophilisation cycles can generate an amorphous sucrose glass. Amorphous glass can form in the presence of high plasmid DNA concentrations and also in the presence of all the described excipients. However, as it was unclear whether or not some crystalline material was present in the samples or had been formed during the DSC analysis itself then further samples were analysed by the technique of polarised light microscopy to determine the amorphous / crystalline nature of the samples.

### Analysis of lyophilised, plasmid DNA formulations by polarized light microscopy.

The lyophilised plasmid DNA / sucrose, (± excipients), samples prepared for DSC analysis, described above, were subject to analysis by polarized light microscopy. Control samples were prepared of simply 40% sucrose, lyophilised for 1 hour and 24 hour cycles and crystalline samples of sucrose and the major solid excipients: methionine, Tris HCl and EDTA were also analysed. This was for comparison and to note the appearance of any crystalline material present in the formulations. The analysis was performed on a Zeiss STD16-444111 polarised light microscope with samples mounted in immersion oil and covered.

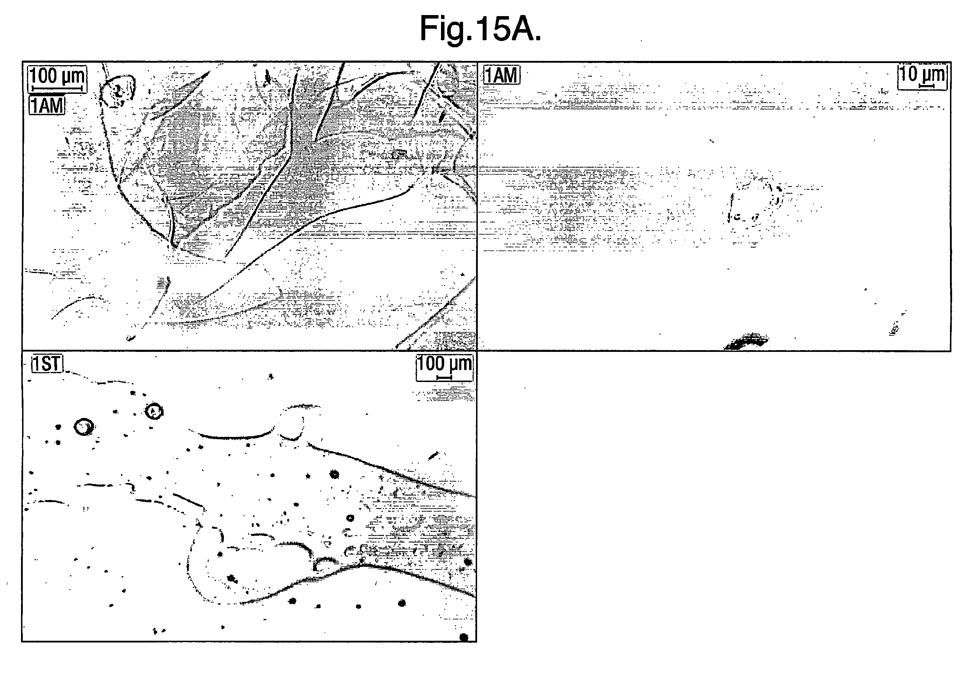

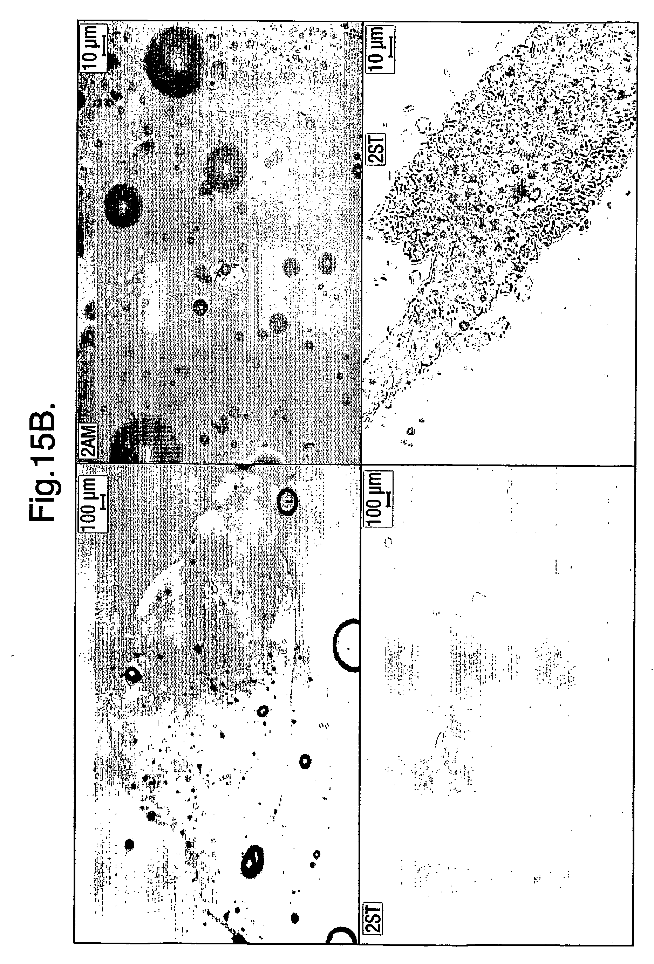

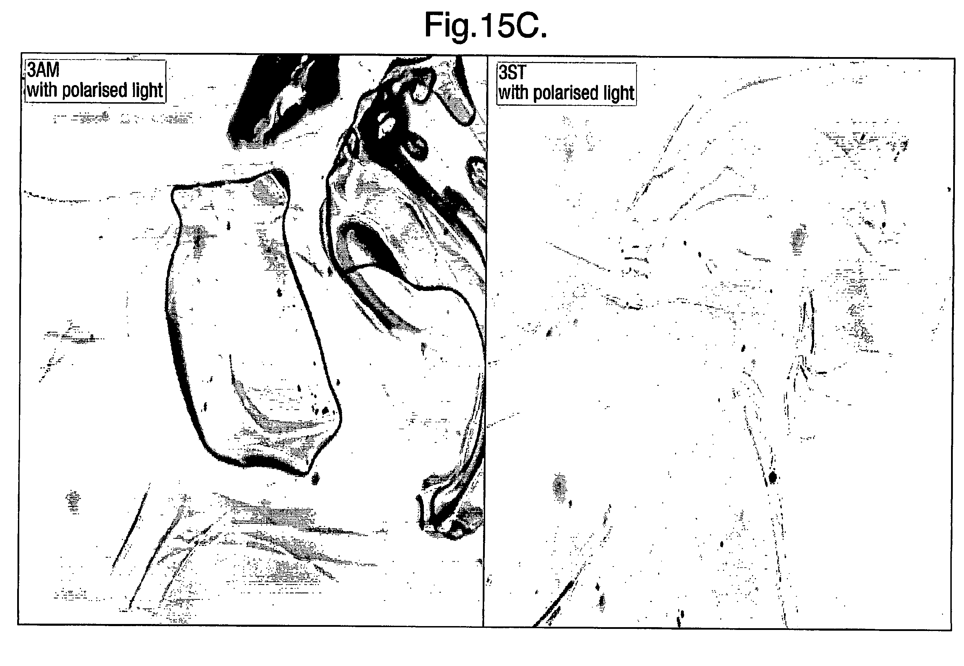

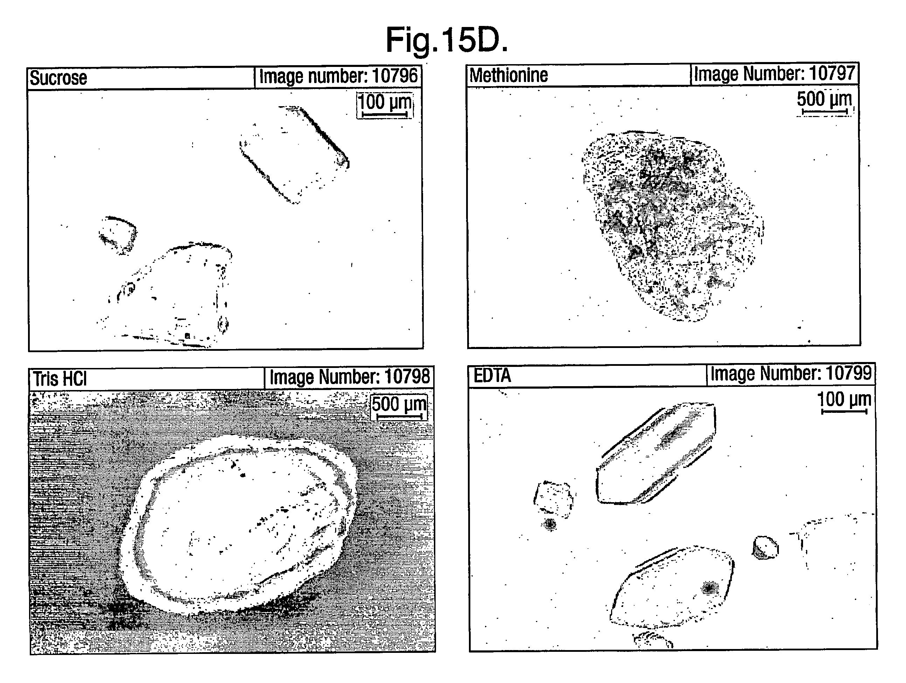

The data is shown in FIG 15 where all formulations contain plasmid DNA, (10mg/ml), in 40% sucrose. Fig 15A: formulations also contain: 100mM TrisHCl pH8.0, 1mM EDTA, 10mM methionine and 2.9% ethanol, Fig15C: only contains 40% sucrose and Fig 15D: shows crystals of the major solid excipient. 1AM, 2AM & 3AM represent a 24 hour lyophilization cycle, whereas 1ST, 2ST & 3ST represent a 1 hour lyophilization cycle.

From Fig. 15C it is clear that both 1 hour and 24 hour lyophilisation cycles performed on 40% sucrose alone generate solely an amorphous glass as expected. From Fig 15B, the addition of plasmid DNA, (10mg/ml) to the 40% sucrose formulation, although it allows largely for the formation of an amorphous glass, does enable the partial formation of some crystalline sucrose (2AM and 2ST samples consist of amorphous material with some evidence for some crystal particles, which could be sucrose). However, from Fig 15A, the addition of the excipients to the DNA / sucrose formulation reduces the amount of crystalline particle formulation in samples lyophilised for 24 hours (1AM, the bulk of the sample consists of sheets of amorphous material. There are few crystalline particles present), and for short lyophilisation cycles of 1 hour, there is no evidence for the formation of crystalline particles, simply an amorphous glass. This suggests that the addition of the described excipients to plasmid DNA in sucrose helps not only to improve DNA release and stability from degradation but also to help preserve the amorphous glass state upon short cycles of lyophilisation.

### Example 14, Demonstration of amorphous glass formation after lyophilization of plasmid DNA, in different sugar lpolyol formulations containing excipients.

To determine if the nature of the polyol / sugar present in the plasmid DNA formulation with excipients, as described above, affected the ability of such formulations to generate an amorphous glass upon lyophilisation, the polyol was varied. A number of similar formulations that differed only in the polyol present were generated, lyophilised and analysed by polarised light microscopy. This was performed in a similar manner to that described in example 13 except that on this occasion an Olympus BX51 polarized light microscope was used.

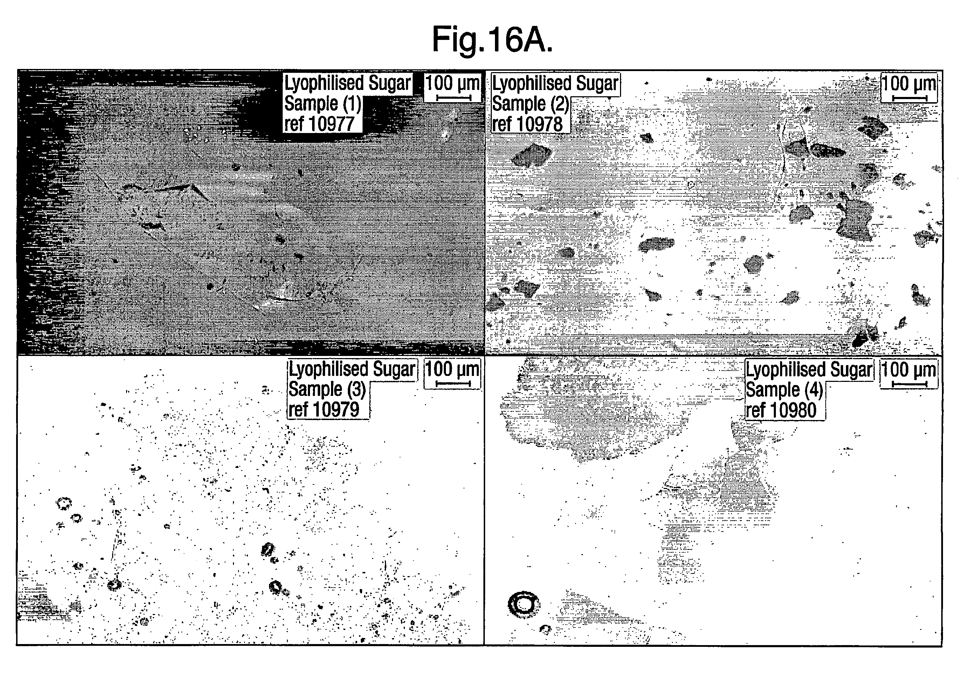

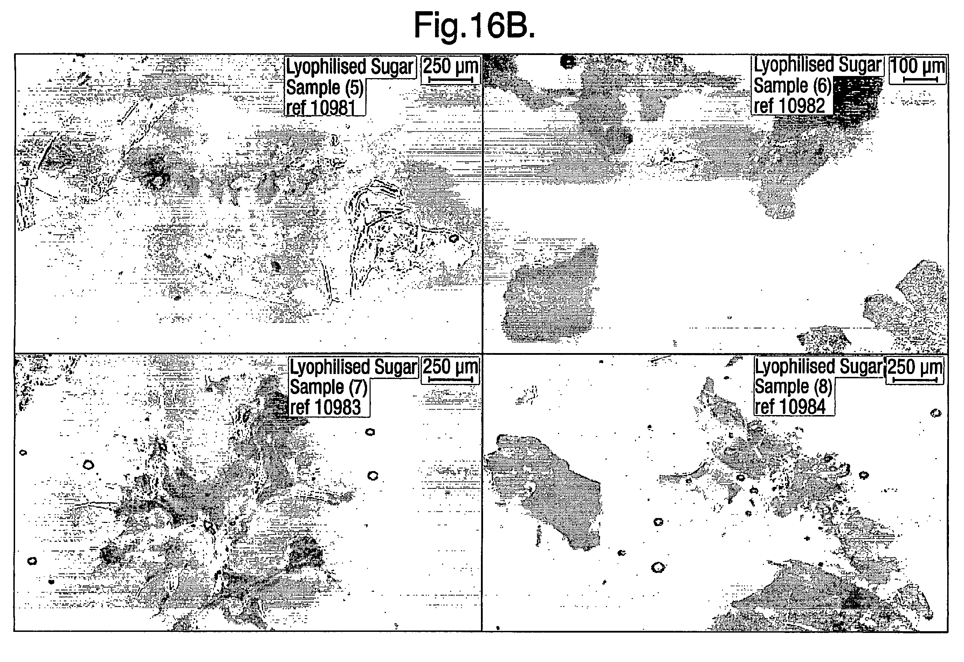

The data is shown in FIG 16 where all formulations contain lyophilisized plasmid DNA, (10mg/ml), and 100mM TrisHCl pH8.0, 1mM EDTA, 10mM methionine and 2.9% ethanol. Fig 16A, sample 1: 40% w/v ficoll, sample 2:20% w/v dextran, sample 3:40% w/v sucrose, sample 4:20% w/v maltotriose. Fig 16B, sample 5:20% w/v lactose, sample 6: 30% w/v maltose, sample 7: 40% w/v glucose, sample 8:40% w/v trehalose. Note that all the samples described and all the formulations analysed formed an amorphous glass with little or no evidence of crystalline material being present. Note that the formulation described as sample 2, containing 20% w/v dextran, was subsequently shown to have precipitated the plasmid DNA out of solution, by agarose gel electrophoretic analysis, (data not shown), and would therefore not be a preferred formulation. This data demonstrates that plasmid DNA plus the described excipients can be maintained, when lyophilised, in an amorhous glass state by a variety of polyols / sugars described in the literature, (Hatley, R & Blair, J., (1999), Journal of Molecular Catalysis B: Enzymatic 7: 11-19.).

## Claims

1. A DNA pharmaceutical agent delivery device having at least one skin-piercing microneedle which comprises a support member coated with a solid reservoir medium containing the DNA pharmaceutical agent, and a stabilising agent that inhibits the degradative effects of free radicals

2. A DNA pharmaceutical agent delivery device as claimed in claim 1 wherein the stabilising agent is one or both of a metal ion chelator and a free radical scavenger.

3. A DNA pharmaceutical agent delivery device as claimed in claim 2 wherein the metal ion chelating agent is selected from the group consisting of inositol hexaphosphate, tripolyphosphate, succinic and malic acid, ethylenediamine tetraacetic acid (EDTA), tris (hydroxymethyl) amino methane (TRIS), Desferal, diethylenetriaminepentaacetic acid (DTPA) and ethylenediamindihydroxyphenylacetic acid (EDDHA).

4. A DNA pharmaceutical agent delivery device as claimed in claim 2 wherein the non-reducing free radical scavenger is selecting from the group consisting of ethanol, methionine or glutathione.

5. A DNA pharmaceutical agent delivery device as claimed in claim 2 wherein the stabilising agent that inhibits the degradative effects of free radicals, is (a) Phosphate buffered ethanol solution in combination with methionine or EDTA, or (b) Tris buffered EDTA in combination with methionine or ethanol (or combinations of methionine and ethanol).

6. A DNA pharmaceutical agent delivery device as claimed in any one of claims 1 to 5, wherein the solid reservoir medium is an amorphous polyol.

7. A DNA pharmaceutical agent delivery device as claimed in claim 6, wherein the polyol is a stabilising polyol.

8. A DNA pharmaceutical agent delivery device as claimed in any one of claims 1 to 7 wherein the solid biodegradable reservoir medium is a sugar.

9. A DNA pharmaceutical agent delivery device as claimed in claim 8 wherein the sugar is selected from lactose, glucose, sucrose, raffinose or trehalose.

10. A DNA pharmaceutical agent delivery device as claimed in any one of claims 1 to 9 wherein the solid reservoir medium is in the form of a glass.

11. A DNA pharmaceutical agent delivery device as claimed in claim 10, wherein the solid reservoir medium is in the form of a sugar glass.

12. A DNA pharmaceutical agent delivery device as claimed in any one of claims 1 to 11 wherein the DNA is supercoiled plasmid DNA

13. A DNA pharmaceutical agent delivery device as claimed in claim 12, wherein the supercoiled plasmid DNA is stabilised such that after storage at 37°C for 4 weeks greater than 50% of the DNA remains in its supercoiled form.

14. A DNA pharmaceutical agent delivery device as claimed in claim 12, wherein the DNA is stabilised such that when released the ratio of monomer:dimer supercoiled form is within the range of 0.8:1.2.

15. A DNA pharmaceutical agent delivery device as claimed in any one of claims 1 to 14 wherein the solid biodegradable reservoir medium releases the pharmaceutical agent within 24 hours after insertion of the skin-piercing microneedle into the skin.

16. A DNA pharmaceutical agent delivery device as claimed in any one of claims 1 to 15 wherein the skin piercing members are dimensioned to deliver the agent into the dermis.

17. A DNA pharmaceutical agent delivery device as claimed in any one of claims 1 to 15, wherein the skin piercing members are dimensioned to deliver the agent into the epidermis.

18. A DNA pharmaceutical agent delivery device as claimed in any one of claims 1 to 17 wherein the support members are solid needles, microcannulas or microblades.

19. A DNA pharmaceutical agent delivery device as claimed in any one of claims 1 to 18, wherein the device is an electroporation device.

20. A DNA pharmaceutical agent delivery device as claimed in claim 19 wherein the coated support members of the device are the electrodes of the electroporation device.

21. A DNA pharmaceutical agent delivery device as claimed in any one of claims 1 to 20, wherein the pharmaceutical agent is a vaccine.

22. A DNA pharmaceutical agent delivery device as claimed in any one of claims 1 to 21, wherein the solid reservoir medium further comprises a vaccine adjuvant, transfection facilitating agent, DNAase inhibitor or a crystal poisoner.

23. A DNA pharmaceutical agent delivery device as claimed in claim 22, wherein the adjuvant is selected from the group consisting of CpG, a synthetic imidazoquinolines, tucerasol, cytokines, MPL, QS21, QS7 and oil in water emulsions.

24. A process for the preparation of a DNA pharmaceutical agent delivery device as claimed in claim 1, comprising making a solution of DNA pharmaceutical agent, reservoir medium, and stabilising agent that inhibits the degradative effects of free radicals in an solvent, followed by coating the at least one support member with said solution, and removing the solvent to form a solid reservoir medium containing the pharmaceutical agent and agent that inhibits the degradative effects of free radicals.

25. A process for the preparation of a DNA pharmaceutical agent delivery device as claimed in claim 24, wherein the reservoir medium is a sugar.

26. A process for the preparation of a DNA pharmaceutical agent delivery device as claimed in claim 25 wherein the concentration of sugar prior to drying onto the support member is in the range of 20-40% w/v.

27. A process for the preparation of a DNA pharmaceutical agent delivery device as claimed in claim 24, wherein the solvent is demetalated prior to the process.

## Patentansprüche

1. Übertragungsvorrichtung für ein DNA-Pharmazeutikum mit wenigstens einer hautdurchbohrenden Mikronadel, die ein Trägerelement umfaßt, das mit einem festen Reservoirmedium beschichtet ist, das das DNA-Pharmazeutikum und ein Stabilisierungsmittel enthält, das die abbauenden Wirkungen von freien Radikalen inhibiert.

2. Übertragungsvorrichtung für ein DNA-Pharmazeutikum gemäß Anspruch 1, worin das Stabilisierungsmittel eines oder beides von einem Metallionen-Komplexbildner und einem freie Radikalfänger ist.

3. Übertragungsvorrichtung für ein DNA-Pharmazeutikum gemäß Anspruch 2, worin der Metallionen-Komplexbildner ausgewählt ist aus der Gruppe bestehend aus Inositolhexaphosphat, Tripolyphosphat, Bernstein- und Äpfelsäure, Ethylendiamintetraessigsäure (EDTA), Tris(hydroxymethyl)aminomethan (TRIS), Desferal, Diethylentriaminpentaessigsäure (DTPA) und Ethylendiamindihydroxyphenylessigsäure (EDDHA).

4. Übertragungsvorrichtung für ein DNA-Pharmazeutikum gemäß Anspruch 2, worin der nicht-reduzierende freie Radikalfänger ausgewählt ist aus der Gruppe bestehend aus Ethanol, Methionin oder Glutathion.

5. Übertragungsvorrichtung für ein DNA-Pharmazeutikum gemäß Anspruch 2, worin das Stabilisierungsmittel, das die abbauenden Wirkungen von freien Radikalen inhibiert, (a) eine Phosphat-gepufferte Ethanollösung in Kombination mit Methionin oder EDTA oder (b) Tris-gepufferte EDTA in Kombination mit Methionin oder Ethanol (oder Kombinationen von Methionin und Ethanol) ist.

6. Übertragungsvorrichtung für ein DNA-Pharmazeutikum gemäß irgendeinem der Ansprüche 1 bis 5, worin das feste Reservoirmedium ein amorphes Polyol ist.

7. Übertragungsvorrichtung für ein DNA-Pharmazeutikum gemäß Anspruch 6, worin das Polyol ein stabilisierendes Polyol ist.

8. Übertragungsvorrichtung für ein DNA-Pharmazeutikum gemäß irgendeinem der Ansprüche 1 bis 7, worin das feste bioabbaubare Reservoirmedium ein Zucker ist.

9. Übertragungsvorrichtung für ein DNA-Pharmazeutikum gemäß Anspruch 8, worin der Zucker ausgewählt ist aus Lactose, Glucose, Saccharose, Raffinose oder Trehalose.

10. Übertragungsvorrichtung für ein DNA-Pharmazeutikum gemäß irgendeinem der Ansprüche 1 bis 9, worin das feste Reservoirmedium in der Form eines Glases ist.

11. Übertragungsvorrichtung für ein DNA-Pharmazeutikum gemäß Anspruch 10, worin das feste Reservoirmedium in der Form eines Zuckerglases ist.

12. Übertragungsvorrichtung für ein DNA-Pharmazeutikum gemäß irgendeinem der Ansprüche 1 bis 11, worin die DNA eine superspiralisierte Plasmid-DNA ist.

13. Übertragungsvorrichtung für ein DNA-Pharmazeutikum gemäß Anspruch 12, worin die superspiralisierte Plasmid-DNA derart stabilisiert ist, daß nach Lagerung bei 37°C für 4 Wochen mehr als 50 % der DNA in ihrer superspiralisierten Form verbleibt.

14. Übertragungsvorrichtung für ein DNA-Pharmazeutikum gemäß Anspruch 12, worin die DNA derart stabilisiert ist, daß Freisetzung das Verhältnis von monomerer zu dimerer superspiralisierter Form im Bereich von 0,8:1,2 ist.

15. Übertragungsvorrichtung für ein DNA-Pharmazeutikum gemäß irgendeinem der Ansprüche 1 bis 14, worin das feste bioabbaubare Reservoirmedium das Pharmazeutikum innerhalb von 24 Stunden nach Einbringen der hautdurchbohrenden Mikronadel in die Haut freisetzt.

16. Übertragungsvorrichtung für ein DNA-Pharmazeutikum gemäß irgendeinem der Ansprüche 1 bis 15, worin die Hautdurchbohrungselemente dimensioniert sind, um das Pharmazeutikum in die Dermis zu übertragen.

17. Übertragungsvorrichtung für ein DNA-Pharmazeutikum gemäß irgendeinem der Ansprüche 1 bis 15, worin die Hautdurchbohrungselemente dimensioniert sind, um das Pharmazeutikum in die Epidermis zu übertragen.

18. Übertragungsvorrichtung für ein DNA-Pharmazeutikum gemäß irgendeinem der Ansprüche 1 bis 17, worin die Trägerelemente massive Nadeln, Mikrokanülen oder Mikroklingen sind.

19. Übertragungsvorrichtung für ein DNA-Pharmazeutikum gemäß irgendeinem der Ansprüche 1 bis 18, worin die Vorrichtung eine Elektroporationsvorrichtung ist.

20. Übertragungsvorrichtung für ein DNA-Pharmazeutikum gemäß Anspruch 19, worin die beschichteten Trägerelemente der Vorrichtung die Elektroden der Elektroporationsvorrichtung sind.

21. Übertragungsvorrichtung für ein DNA-Pharmazeutikum gemäß irgendeinem der Ansprüche 1 bis 20, worin das Pharmazeutikum ein Impfstoff ist.

22. Übertragungsvorrichtung für ein DNA-Pharmazeutikum gemäß irgendeinem der Ansprüche 1 bis 21, worin das feste Reservoirmedium ferner ein Impfstoffadjuvans, ein Transfektions-ermöglichendes Mittel, einen DNAse-Inhibitor oder ein Kristallgift umfaßt.

23. Übertragungsvorrichtung für ein DNA-Pharmazeutikum gemäß Anspruch 22, worin das Adjuvans ausgewählt ist aus der Gruppe bestehend aus CpG, synthetischen Imidazochinolinen, Tucerasol, Cytokinen, MPL, QS21, QS7 und Öl-in-Wasser-Emulsionen.

24. Verfahren zur Herstellung einer Übertragungsvorrichtung für ein DNA-Pharmazeutikum gemäß Anspruch 1, umfassend das Herstellen einer Lösung aus dem DNA-Pharmazeutikum, dem Reservoirmedium und dem Stabilisierungsmittel, das die abbauenden Wirkungen von freien Radikalen in einem Lösungsmittel inhibiert, gefolgt von Beschichten des wenigstens einen Trägerelements mit der Lösung und Entfernen des Lösungsmittels, um ein festes Reservoirmedium zu bilden, das das Pharmazeutikum und das Mittel, das die abbauenden Wirkungen von freien Radikalen inhibiert, enthält.

25. Verfahren zur Herstellung einer Übertragungsvorrichtung für ein DNA-Pharmazeutikum gemäß Anspruch 24, worin das Reservoirmedium ein Zucker ist.

26. Verfahren zur Herstellung einer Übertragungsvorrichtung für ein DNA-Pharmazeutikum gemäß Anspruch 25, worin die Zuckerkonzentration vor der Trocknung auf dem Trägerelement im Bereich von 20 bis 40 % G/V ist.

27. Verfahren zur Herstellung einer Übertragungsvorrichtung für ein DNA-Pharmazeutikum gemäß Anspruch 24, worin das Lösungsmittel vor dem Verfahren entmetallisiert wird.

## Revendications

1. Dispositif de délivrance d'un agent pharmaceutique à base d'ADN comportant au moins une micro-aiguille pour percer la peau qui comprend un élément de support enrobé d'un milieu de réservoir solide contenant l'agent pharmaceutique à base d'ADN et un agent stabilisant qui inhibe les effets dégradants des radicaux libres.

2. Dispositif de délivrance d'un agent pharmaceutique à base d'ADN selon la revendication 1, dans lequel l'agent stabilisant est l'un ou l'autre ou les deux d'un chélateur d'ions métalliques et d'un piégeur de radicaux libres.

3. Dispositif de délivrance d'un agent pharmaceutique à base d'ADN selon la revendication 2, dans lequel l'agent de chélation des ions métalliques est choisi dans le groupe constitué d'hexaphosphate d'inositol, de tripolyphosphate, d'acide succinique et malique, d'acide éthylène-diamine-tétra-acétique (EDTA), de tris (hydroxyméthyl) amino méthane (TRIS), de Desféral, d'acide diéthylène-triamine-penta-acétique (DTPA) et d'acide éthylène-diamine-dihydroxy-phénylacétique (EDDHA).

4. Dispositif de délivrance d'un agent pharmaceutique à base d'ADN selon la revendication 2, dans lequel le piégeur de radicaux libres non réducteurs est choisi dans le groupe constitué d'éthanol, de méthionine ou de glutathion.

5. Dispositif de délivrance d'un agent pharmaceutique à base d'ADN selon la revendication 2, dans lequel l'agent stabilisant qui inhibe les effets dégradants des radicaux libres, est (a) une solution d'éthanol tamponnée par du phosphate en combinaison avec de la méthionine ou de l'EDTA, ou (b) de l'EDTA tamponné par du Tris en combinaison avec de la méthionine ou de l'éthanol (ou des combinaisons de méthionine et d'éthanol).

6. Dispositif de délivrance d'un agent pharmaceutique à base d'ADN selon l'une quelconque des revendications 1 à 5, dans lequel le milieu du réservoir solide est un polyol amorphe.

7. Dispositif de délivrance d'un agent pharmaceutique à base d'ADN selon la revendication 6, dans lequel le polyol est un polyol stabilisant.

8. Dispositif de délivrance d'un agent pharmaceutique à base d'ADN selon l'une quelconque des revendications 1 à 7, dans lequel le milieu du réservoir solide biodégradable est un sucre.

9. Dispositif de délivrance d'un agent pharmaceutique à base d'ADN selon la revendication 8, dans lequel le sucre est choisi parmi le lactose, le glucose, le saccharose, le raffinose ou le tréhalose.

10. Dispositif de délivrance d'un agent pharmaceutique à base d'ADN selon l'une quelconque des revendications 1 à 9, dans lequel le milieu du réservoir solide est sous la forme d'un verre.

11. Dispositif de délivrance d'un agent pharmaceutique à base d'ADN selon la revendication 10, dans lequel le milieu du réservoir solide est sous la forme d'un verre de sucre.

12. Dispositif de délivrance d'un agent pharmaceutique à base d'ADN selon l'une quelconque des revendications 1 à 11, dans lequel l'ADN est de l'ADN plasmidique surenroulé.

13. Dispositif de délivrance d'un agent pharmaceutique à base d'ADN selon la revendication 12, dans lequel l'ADN plasmidique surenroulé est stabilisé de telle manière qu'après un stockage à 37°C pendant 4 semaines, plus de 50 % de l'ADN demeure sous sa forme surenroulée.

14. Dispositif de délivrance d'un agent pharmaceutique à base d'ADN selon la revendication 12, dans lequel l'ADN est stabilisé de telle manière que lorsqu'il est libéré le rapport de la forme surenroulée monomère/dimère se situe dans la plage de 0,8/1,2.

15. Dispositif de délivrance d'un agent pharmaceutique à base d'ADN selon l'une quelconque des revendications 1 à 14, dans lequel le milieu du réservoir solide biodégradable libère l'agent pharmaceutiques en 24 heures après l'insertion de la micro-aiguille perçant la peau, dans la peau.

16. Dispositif de délivrance d'un agent pharmaceutique à base d'ADN selon l'une quelconque des revendications 1 à 15, dans lequel les éléments perçant la peau sont dimensionnés pour délivrer l'agent dans le derme.

17. Dispositif de délivrance d'un agent pharmaceutique à base d'ADN selon l'une quelconque des revendications 1 à 15, dans lequel les éléments perçant la peau sont dimensionnés pour délivrer l'agent dans l'épiderme.

18. Dispositif de délivrance d'un agent pharmaceutique à base d'ADN selon l'une quelconque des revendications 1 à 17, dans lequel les éléments de support sont des aiguilles solides, des microcanules ou des microlames.

19. Dispositif de délivrance d'un agent pharmaceutique à base d'ADN selon l'une quelconque des revendications 1 à 18, dans lequel le dispositif est un dispositif d'électroporation.

20. Dispositif de délivrance d'un agent pharmaceutique à base d'ADN selon la revendication 19, dans lequel les éléments de support enrobés du dispositif sont les électrodes du dispositif d'électroporation.

21. Dispositif de délivrance d'un agent pharmaceutique à base d'ADN selon l'une quelconque des revendications 1 à 20, dans lequel l'agent pharmaceutique est un vaccin.

22. Dispositif de délivrance d'un agent pharmaceutique à base d'ADN selon l'une quelconque des revendications 1 à 21, dans lequel le milieu du réservoir solide comprend en outre un adjuvant vaccinal, un agent facilitant la transfection, un inhibiteur d'ADNase ou un empoisonneur de cristaux.

23. Dispositif de délivrance d'un agent pharmaceutique à base d'ADN selon la revendication 22, dans lequel l'adjuvant est choisi dans le groupe constitué de CpG, d'imidazoquinoléines synthétiques, de tucérasol, de cytokines, de MPL, de QS21, de QS7 et d'émulsions huile dans l'eau.

24. Procédé de préparation d'un dispositif de délivrance d'un agent pharmaceutique à base d'ADN selon la revendication 1, comprenant la fabrication d'une solution d'agent pharmaceutique à base d'ADN, du milieu du réservoir et d'un agent stabilisant qui inhibe les effets dégradants des radicaux libres dans un solvant, ceci suivi de l'enrobage d'au moins un élément de support avec ladite solution et l'élimination du solvant pour former un milieu de réservoir solide contenant l'agent pharmaceutique et l'agent qui inhibe les effets dégradants des radicaux libres.

25. Procédé de préparation d'un dispositif de délivrance d'un agent pharmaceutique à base d'ADN selon la revendication 24, dans lequel le milieu du réservoir est un sucre.

26. Procédé de préparation d'un dispositif de délivrance d'un agent pharmaceutique à base d'ADN selon la revendication 25, dans lequel la concentration du sucre avant le séchage sur l'élément de support se situe dans la plage de 20 à 40 % p/v.

27. Procédé de préparation d'un dispositif de délivrance d'un agent pharmaceutique à base d'ADN selon la revendication 24, dans lequel le solvant est démétallisé avant le procédé.
